# EUROPEAN PATENT APPLICATION

(11) **EP 3 689 382 A1**
(43) Date of publication of application: **05.08.2020**
(21) Application number: 18862746.7
(22) Date of filing: 28.09.2018
(51) Int. Cl.: A61K 47/64, A61K 47/60, A61K 47/68, A61K 47/54, A61K 38/28, C07K 14/62

(54) **LONG-ACTING SINGLE-CHAIN INSULIN ANALOG AND CONJUGATE THEREOF**

(30) Priority: 28.09.2017 KR 20170126476
(71) Applicant: Hanmi Pharm. Co., Ltd., Hwaseong-si, Gyeonggi-do 18536 (KR)
(72) Inventor: HEO, Yong Ho, Hwaseong-si Gyeonggi-do 18469 (KR); OH, Euh Lim, Hwaseong-si Gyeonggi-do 8469 (KR)
(74) Representative: Mewburn Ellis LLP
(86) International application number: PCT/KR2018/011557
(87) International publication number: WO 2019/066570

(57) **Abstract**

The present invention relates to a long-acting single-chain insulin analog, a conjugate thereof, and uses of the same. In addition, the present invention relates to a method for preparing a long-acting single-chain insulin analog and a conjugate thereof.

## Description

### [Technical Field]

The present invention relates to a long-acting single-chain insulin analog, a conjugate thereof, and uses of the same. In addition, the present invention relates to a method for preparing a long-acting single-chain insulin analog and a conjugate thereof.

### [Background Art]

Insulin is a polypeptide hormone composed of 51 amino acids secreted by beta cells of the pancreas, and is involved in the regulation of blood glucose in animals. Insulin consists of A chain and B chain, which are linked by a disulfide bond, and C-peptide is removed (hydrolyzed) *in vivo* by proteases in proinsulin to produce insulin from proinsulin.

Insulin formulations are a blood glucose regulator administered to Type I diabetes patients who have congenital defects in insulin production; Type II diabetes patients where blood sugar levels rise secondarily due to insulin resistance, lack of insulin secretion, *etc.,* and who are poorly controlled by oral diabetes medication or whom oral diabetes medication is contraindicated; or gestational diabetes patients.

In the early years, the insulin of cows and pigs was used after purification, but the development of genetic engineering and DNA manipulation technique enabled the mass production of human insulin using bacteria and yeast (European Patent Application Publication No. EP 0055945), and it is possible to produce insulin analogs, *etc.* by modifying human insulin to improve action time, *etc.*

However, insulin, like other protein and peptide hormones, has a very short half-life in the body, which makes it difficult to exhibit a therapeutic effect continuously, and in order to show the effect, it is necessary to continuously repeat the administration. In addition, protein and peptide drugs are administered to patients in the form of injections most of the time, which are frequently injected to maintain blood levels of bioactive peptides, and it causes tremendous pain in the patient. Therefore, studies have been focused on the development of several protein formulations and chemical modifications thereof so as to increase the therapeutic effects and improve the quality of life of patients by reducing the frequency of administration via the increase of the *in vivo* half-life of these proteins.

Single-chain insulin analogs can be used in a method to solve the problems of duration and low blood glucose levels of insulin formulations. Single-chain insulin can be used to control half-life and potency by preparing and using it as a single chain, instead of being prepared in the form where the existing A chain and B chain are linked by a disulfide bond. Single-chain insulin analogs can be linked in the order of B chain and A chain or in the order of A chain and B chain, and a peptide may be inserted there between as a linker when linking each chain.

Over the last few decades, many types of single-chain insulin analogs have been made. These analogs linked the C-terminus of B chain and the N-terminus of A chain with linkers consisting of peptides or compounds of various lengths. However, the single-chain insulin analog, in which LysB29 located at the C- terminus of B chain was directly linked to GlyA1 at the N-terminus of A chain, showed biological inactivity (Derewenda, U. et al, J. Mol. Biol., 1991, 220: 425-433).

Another method for improving the duration of insulin formulations is the use of proinsulin, a precursor of insulin. Proinsulin itself is a weak insulin agonist that has a lower activity than insulin but has a longer half-life than insulin.

In proinsulin, unlike insulin, B chain and A chain are linked by a C-peptide, which plays an important role in maintaining the stability of insulin, and it has a longer blood half-life than insulin. Thus, many researchers have made various attempts to develop proinsulin into long-acting insulin. However, the amino-terminal glycine residue of the A chain, which plays an important role in its activity, is hidden by a C-peptide, thus resulting in a lower activity than insulin *(*William F et al., The Journal of Biological Chemistry, 1992, 267: 419-425*),* and it has not been developed as a drug yet.

Most proteins that are produced by utilizing a genetic recombination technique are produced in a form in which methionine is bound to the N-terminus of the protein. As one of the existing insulin preparation methods for removing methionine, which is not a necessary amino acid, there is a method of using trypsin, which is a serine protease (European Patent Application Publication No. EP 1926749). However, since a trypsin recognition sequence is present in proinsulin, this approach is not suitable for the preparation of single-chain insulin analogs. As an alternative method, there is a method of removing methionine at the N-terminus of proteins, using cyanogen bromide (CNBr) which has been widely used traditionally, but the extreme toxicity of CNBr, the increased complexity of manufacturing processes, low production yields, *etc.* have been raised as problems. Accordingly, there is a need for insulin formulations that can maintain increased duration and activity.

### [Disclosure]

### [Technical Problem]

An object of the present invention is to provide a single-chain insulin analog.

Another object of the present invention is to provide a single-chain insulin analog conjugate, wherein a single-chain insulin analog and a material capable of increasing an *in vivo* half-life of the same are linked.

Still another object of the present invention is to provide a method for preparing a single-chain insulin analog conjugate, comprising linking the single-chain insulin analog and a material capable of increasing an *in vivo* half-life of the same.

Still another object of the present invention is to provide a long-acting single-chain insulin formulation with enhanced *in vivo* duration and stability, comprising the single-chain insulin analog and/or a single-chain insulin analog conjugate.

Still another object of the present invention is to provide a pharmaceutical composition for preventing or treating diabetes, comprising the single-chain insulin analog and/or single-chain insulin analog conjugate.

Still another object of the present invention is to provide a method for preventing or treating diabetes, comprising administering the pharmaceutical composition to a subject in need thereof.

Still another object of the present invention is to provide use of the single-chain insulin analog conjugate or the single-chain insulin analog for the preparation of a drug for preventing or treating diabetes.

Still another object of the present invention is to provide use of the single-chain insulin analog conjugate or the single-chain insulin analog for preventing or treating diabetes.

Still another object of the present invention is to provide an isolated nucleic acid encoding the single-chain insulin analog, a recombinant expression vector comprising the nucleic acid, and a transformant comprising the recombinant expression vector.

Still another object of the present invention is to provide a method for preparing a single-chain insulin analog using the transformant.

### [Technical Solution]

An aspect of the present invention is to provide a single-chain insulin analog conjugate having Chemical Formula 1 below:

[Chemical Formula 1] X-Y-Z-La-F

wherein:
X is native insulin A chain, B chain, or an analog thereof,
Y is native insulin C-peptide or an analog thereof,
Z is native insulin B chain, A chain, or an analog thereof,
L is a linker,
A is 0 or a natural number, with the proviso that when a is 2 or more, each L is independent of each other,
F is a material capable of increasing an *in vivo* half-life of an insulin analog,
with the proviso that cases where X, Y, and Z are a B chain, C-peptide, and A chain of native insulin, respectively, or an A chain, C-peptide, and B chain of native insulin, respectively, are excluded, and
X-Y-Z forms a single-chain insulin analog.

A specific aspect of the present invention provides a single-chain insulin analog conjugate, wherein the single-chain insulin analog is an analog, a variant, or a fragment thereof, which is modified by at least one method selected from substitution, addition and modification of at least one amino acid, and change in arrangement of order (the order of insulin B chain, C-peptide, and A chain, *etc*.), compared to native proinsulin.

Another specific aspect of the present invention provides a single-chain insulin analog conjugate, wherein the single-chain insulin analog is a single-chain insulin analog in which X, Y, and Z are each linked by a linker.

Still another specific aspect of the present invention provides a single-chain insulin analog conjugate, wherein the analog of the C-peptide is an analog of the C-peptide in which at least one amino acid selected from the group consisting of the 1^{st} amino acid, the 2^{nd} amino acid, the 34^{th} amino acid, and the 35^{th} amino acid of native insulin C-peptide is substituted with another amino acid or deleted.

Still another specific aspect of the present invention provides a single-chain insulin analog conjugate, wherein a material capable of increasing an *in vivo* half-life of an insulin analog is selected from the group consisting of polyethylene glycol, fatty acid, cholesterol, albumin and a fragment thereof, an albumin-binding material, a polymer of repeating units of a specific amino acid sequence, an antibody, an antibody fragment, an FcRn-binding material, *in vivo* connective tissue, nucleotide, fibronectin, transferrin, saccharide, and a polymer.

Still another specific aspect of the present invention provides a single-chain insulin analog conjugate, wherein L is selected from the group consisting of peptide, polyethylene glycol, fatty acid, saccharide, polymer, low molecular weight compound, nucleotide, and a combination thereof.

Still another specific aspect of the present invention is to provide a single-chain insulin analog conjugate, wherein X-Y-Z and F are linked via L by a covalent chemical bond, a non-covalent chemical bond, or a combination thereof.

Still another specific aspect of the present invention provides a single-chain insulin analog conjugate, wherein a polymer is selected from the group consisting of polypropylene glycol, ethylene glycol-propylene glycol copolymer, polyoxyethylated polyol, polyvinyl alcohol, polysaccharide, dextran, polyvinyl ethyl ether, biodegradable polymer, lipid polymer, chitin, hyaluronic acid, oligonucleotide, and a combination thereof.

Still another specific aspect of the present invention provides a single-chain insulin analog conjugate, wherein the FcRn binding material is an immunoglobulin Fc region.

Still another specific aspect of the present invention is to provide a single-chain insulin analog conjugate, wherein the immunoglobulin Fc region is aglycosylated

Still another specific aspect of the present invention is to provide a single-chain insulin analog conjugate, wherein the immunoglobulin Fc region is composed of 1 to 4 domains selected from the group consisting of CH1, CH2, CH3, and CH4 domains.

Still another specific aspect of the present invention is to provide a single-chain insulin analog conjugate, wherein the immunoglobulin Fc region is an Fc region derived from IgG, IgA, IgD, IgE, or IgM.

Still another specific aspect of the present invention is to provide a single-chain insulin analog conjugate, wherein each domain of the immunoglobulin Fc region is a hybrid of domains with different origins derived from an immunoglobulin selected from the group consisting of IgG, IgA, IgD, IgE, and IgM.

Still another specific aspect of the present invention is to provide a single-chain insulin analog conjugate, wherein the immunoglobulin Fc region is a dimer or multimer consisting of single-chain immunoglobulins composed of domains of the same origin.

Still another specific aspect of the present invention is to provide a single-chain insulin analog conjugate, wherein the immunoglobulin Fc region is an IgG4 Fc region.

Still another specific aspect of the present invention is to provide a single-chain insulin analog conjugate, wherein the immunoglobulin Fc region is a human aglycosylated IgG4 Fc region.

Another aspect of the present invention provides a single-chain insulin analog having Chemical Formula 2 below:

[Chemical Formula 2] X-Y-Z

wherein,
X is native insulin A chain, B chain, or an analog thereof,
Y is native insulin C-peptide or an analog thereof,
Z is native insulin B chain, A chain, or an analog thereof, and
with the proviso that cases where X, Y, and Z are B chain, C-peptide, and A chain of native insulin, respectively, or A chain, C-peptide, and B chain of native insulin, respectively, are excluded.

Still another specific aspect of the present invention provides a single-chain insulin analog, wherein the analog of the C-peptide is an analog of the C-peptide in which at least one amino acid selected from the group consisting of the 1^{st} amino acid, the 2^{nd} amino acid, the 34^{th} amino acid, and the 35^{th} amino acid of native insulin C-peptide is substituted with another amino acid or deleted.

Still another specific aspect of the present invention provides a method for preparing the single-chain insulin analog conjugate, comprising linking the single-chain insulin analog to a material capable of increasing the *in vivo* half-life thereof.

Still another specific aspect of the present invention provides a method for preparing the single-chain insulin analog conjugate, wherein the single-chain insulin analog and the material capable of increasing the *in vivo* half-life thereof are linked via a linker.

Still another specific aspect of the present invention provides a method for preparing the single-chain insulin analog conjugate, wherein the linker is a non-peptidyl linker having a reactive group selected from the group consisting of an aldehyde group, propionaldehyde group, butyl aldehyde group, a maleimide group, and a succinimide derivative.

Still another specific aspect of the present invention provides a method for preparing the single-chain insulin analog conjugate, wherein the succinimide derivative is succinimidyl propionate, methylpropionate, succinimidyl butanoate, succinimidyl methylbutanoate, succinimidyl valerate, succinimidyl carboxymethyl, N-hydroxysuccinimide, or succinimidyl carbonate.

Still another specific aspect of the present invention analog is obtained by a method comprising (a) and (b) below:
(a) expressing a single-chain insulin analog in a form, in which a peptide consisting of 5 to 20 amino acids comprising a protease cleavage site is fused to an N-terminus of the single-chain insulin analog; and
(b) removing the peptide fused to the single-chain insulin analog.

Still another specific aspect of the present invention provides a long acting single-chain insulin formulation with enhanced *in vivo* duration and stability, comprising the single-chain insulin analog conjugate; or the single-chain insulin analog.

Still another specific aspect of the present invention provides a pharmaceutical composition for preventing or treating diabetes, comprising the single-chain insulin analog conjugate; or the single-chain insulin analog.

Still another specific aspect of the present invention provides a method for preventing or treating diabetes, comprising administering the pharmaceutical composition to a subject in need thereof.

Still another aspect of the present invention provides use of the single-chain insulin analog conjugate or the single-chain insulin analog for the preparation of a drug for preventing or treating diabetes.

Still another aspect of the present invention provides use of the single-chain insulin analog conjugate or the single-chain insulin analog for preventing or treating diabetes.

Still another aspect of the present invention provides an isolated nucleic acid encoding the single-chain insulin analog, a recombinant expression vector comprising the nucleic acid, and a transformant comprising the recombinant expression vector.

Still another aspect of the present invention provides a method for preparing a single-chain insulin analog using the transformant.

Still another aspect of the present invention provides a method for preparing the single-chain insulin analog, comprising :
a) expressing a single-chain insulin analog by culturing a transformant comprising a nucleic acid encoding the single-chain insulin analog; and
b) isolating and purifying the expressed single-chain insulin analog.

### [Advantageous Effect]

The single-chain insulin analog and a conjugate thereof according to the present invention can maintain a stable hypoglycemic effect *in vivo,* and have significantly increased blood half-life, thereby improve the administration convenience and side effects, and has an effect of simplifying the preparing process.

### [Brief Description of Drawings]

FIG. 1 is a schematic diagram of gene amplification of a single-chain insulin analog.

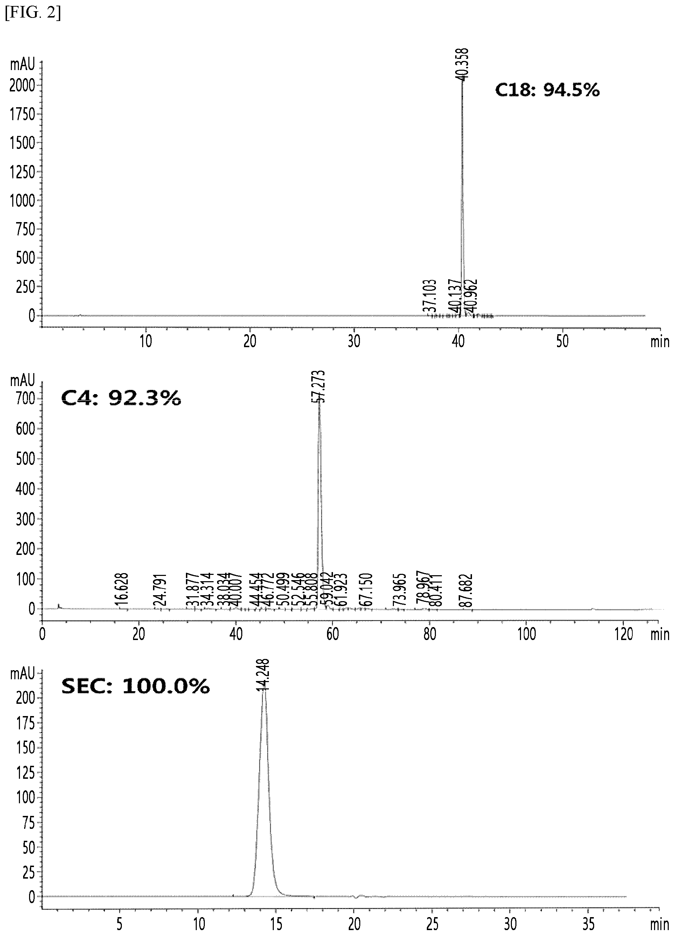

FIG. 2 is the result of purity analysis of a single-chain insulin analog after its purification (C18, C4: RP-HPLC; SEC: SE-HPLC).
FIG. 3 is the result of purity analysis of a single-chain insulin analog conjugate after its purification (SEC: SE-HPLC; IEX: IE-HPLC).
FIG. 4 is the result of confirming the glucose uptake of a single-chain insulin analog conjugate.
FIG. 5 is the result of confirming the pharmacokinetics of a single-chain insulin analog conjugate.

### [Best Mode]

Detailed description for carrying out the present invention is as follows. Meanwhile, each of the descriptions and embodiments disclosed herein may also apply to each of the other descriptions and embodiments. That is, all combinations of the various elements disclosed herein are within the scope of the present invention. In addition, the scope of the present invention is not limited by the specific description described below.

An aspect of the present invention provides a single-chain insulin analog with reduced insulin receptor binding affinity compared to native insulin.

As used herein, the term "single-chain insulin analog" refers to an insulin analog that has *in vivo* blood glucose lowering properties and in which the A chain and B chain of insulin are linked by one chain. For the purposes of the present invention, the single-chain insulin analog is preferably a material having reduced insulin receptor binding affinity and *in vitro* activity compared to native insulin.

Native insulin is a hormone secreted by the pancreas, which generally promotes the glucose uptake in cells, inhibits breakdown of fat to regulates blood glucose levels in the body. For insulin, the form of proinsulin, which does not have a glycemic control function, is processed and becomes insulin with a glycemic control function. The insulin in the body is expressed in the form of proinsulin of the B chain-C peptide-A chain, undergoes enzymatic treatment, and exists in the form of insulin having two polypeptide chains of A chain and B chain , in which each chain includes 21 and 30 amino acid residues, respectively, are linked by two disulfide bridges. The A chain, B chain, and C-peptide of native proinsulin may include amino acid sequences of SEQ ID NOS: 1 to 3, respectively, but are not limited thereto.
A chain: 
Gly-Ile-Val-Glu-Gln-Cys-Cys-Thr-Ser-Ile-Cys-Ser-Leu-Tyr-Gln-Leu-Glu-Asn-Tyr- Cys-
Asn (SEQ ID NO: 1)
B chain: 
Phe-Val-Asn-Gln-His-Leu-Cys-Gly-Ser-His-Leu-Val-Glu-Ala-Leu-Tyr-Leu-Val-Cys -
Gly-Glu-Arg-Gly-Phe-Phe-Tyr-Thr-Pro-Lys-Thr (SEQ ID NO: 2)
C-peptide:

Arg-Arg-Glu-Ala-Glu-Asp-Leu-Gln-Val-Gly-Gln-Val-Glu-Leu-Gly-Gly-Gly-Pro-Gl y-
Ala-Gly-Ser-Leu-Gln-Pro-Leu-Ala-Leu-Glu-Gly-Ser-Leu-Gln-Lys-Arg (SEQ ID NO: 3)

In addition, the single-chain insulin analog of the present invention may be a proinsulin analog, but is not limited thereto. The proinsulin analog refers to wild-type or native proinsulin that is arranged in the order of B chain, C-peptide, and A chain, and peptides where the arrangement of A chain, B chain, and C-peptide is different by one or more. Specifically, a proinsulin analog may have an arrangement of A chain, C-peptide, and B chain, but is not limited thereto.

A single-chain insulin analog in the present invention can be used interchangeably with a proinsulin analog.

The amino acid sequence of each of the A chain, B chain, and C-peptide of the proinsulin analog of the present invention may be either a wild-type sequence or a sequence in which one or more amino acids, either native or not native, are added, substituted, or deleted in the wild-type sequence, but is not limited thereto.

The proinsulin analog of the present invention is at least 80%, specifically at least 90%, more specifically, at least 91%, 92%, 93%, 94%, 95%, 96%, 97%, 98%, or 99% homologous to the A chain, B chain, and C-peptide of native proinsulin respectively, and may be in a form where some groups of amino acid residues may be chemically substituted (*e.g.;* alpha-methylation, alpha-hydroxylation), deletion (*e.g.;* deamination), or modified (*e.g.;* N-methylation), and may include all of the peptides having the function of regulating blood glucose levels in the body, but is not limited thereto.

The term "homology" is intended to represent a degree of similarity to the amino acid sequence of a wild-type protein or a nucleotide sequence encoding the same, and sequences having the amino acid sequence or the base sequence and sequences having at least the same percentage as above of the present invention are also included in the present invention. While such homology can be determined by comparing two sequences by the naked eye, it can also be determined using bioinformatics algorithm that analyzes the degree of homology by arranging the sequences to be compared side by side. The homology between the two amino acid sequences can be expressed as a percentage. Useful automated algorithms are available in the GAP, BESTFIT, FASTA, and TFASTA computer software modules of Wisconsin Genetics Software Package (Genetics Computer Group, Madison, WI, USA). The automated arrangement algorithms in the module include Needleman & Wunsch, Pearson & Lipman, and Smith & Waterman sequence arrangement algorithms. Algorithms and homology determinations for other useful arrangements are automated in software including FASTP, BLAST, BLAST2, PSIBLAST, and CLUSTAL W.

The single-chain insulin analog of the present invention is a peptide having a blood glucose control function *in vivo,* and this peptide includes proinsulin agonists, derivatives, fragments, variants, *etc.,* but is not limited thereto.

The proinsulin agonist of the present invention refers to a material that binds to the *in vivo* receptor of insulin and exhibits the same biological activity as insulin regardless of the structure of proinsulin.

The proinsulin fragment of the present invention refers to a form in which one or more amino acids are added to or deleted from proinsulin, and the added amino acids may be amino acids (e.g.; D-type amino acids) that do not exist in nature, and this proinsulin fragment has a blood glucose control function in the body.

The proinsulin variant of the present invention refers to a peptide having a blood glucose control function in the body, as a peptide having at least one amino acid sequence different from that of proinsulin.

The method for preparing used in the proinsulin agonists, derivatives, fragments, and variants of the present invention may be used independently or in combination. For example, peptides having a blood glucose control function in the body having one or more amino acid sequences different from each other and deaminated to an amino acid residue at the N-terminus are included.

In one specific embodiment, the single-chain insulin analog may be produced through a recombinant method or may be produced by a Solid phase synthesis method.

In one specific embodiment, the single-chain insulin analog of the present invention may be that native insulin A chain or an analog thereof; native insulin C-peptide or an analog thereof; and B chain or an analog thereof are linked directly or by a linker in various orders.

In one specific embodiment, the single-chain insulin analog of the present invention may be an analog, a variant, or a fragment thereof, which is modified by at least one method selected from substitution, addition and modification of at least one amino acid, and change in arrangement of order (order of B chain, C-peptide, and A chain, *etc*.), compared to native proinsulin.

In one specific embodiment, the single-chain insulin analog of the present invention may be that native insulin A chain or an analog thereof; native insulin C-peptide or an analog thereof; native insulin C-peptide or an analog thereof; and native insulin B-chain or an analog thereof are linked in the above order, but the order is not limited thereto.

In one specific embodiment, the single-chain insulin analog of the present invention may comprise C-peptide or an analog thereof. C-peptide (connecting peptide) is a peptide consisting of 35 amino acids, connecting B chain and A chain in proinsulin. While C-peptide is removed during the biosynthesis of insulin, when it is not removed and present in insulin, it lowers the *in vitro* activity of insulin compared to the native type. The C-peptide comprised in the single-chain insulin analog of the present invention may be an analog in which an amino acid is deleted, substituted, and/or inserted into native C-peptide.

In one specific embodiment, the analog of the C-peptide may be an analog of the C-peptide in which at least one amino acid selected from the group consisting of the 1^{st} amino acid, the 2^{nd} amino acid, the 34^{th} amino acid, and the 35^{th} amino acid of native insulin C-peptide is substituted with another amino acid or deleted, but the analog is not limited thereto. More specifically, the analog of the C-peptide may be an analog of the C-peptide in which the 1^{st} and the 2^{nd} arginine, the 34^{th} lysine residue, and the 35^{th} arginine residue of the native insulin C-peptide are removed, thereby being capable of increasing the production efficiency upon production of a single-chain insulin analog, but the analog is not limited thereto.

In one specific embodiment, the single-chain insulin analog of the present invention may be that native insulin A chain; the analog of the C-peptide; and native insulin B chain are linked in the above order, and specifically, may have an amino acid sequence of SEQ ID NO: 19, but single-chain insulin analog is not limited thereto.

In one specific embodiment, the single-chain insulin analog of the present invention may be a fusion in which a peptide is fused to the N-terminus of a single-chain insulin analog. The peptide fused to the N-terminus of single-chain insulin contributes to an increase in the expression yield of the single-chain insulin analog, and has a protease (for example, trypsin) cleavage site so that it is efficiently removed during the production of a single-chain insulin analog. The peptide to be added to the N-terminus of the single-chain insulin analog is not limited as long as it contributes to an increase in the expression yield of single-chain insulin analog and it comprises a protease cleavage site, and specifically, it may be composed of 5 to 20 amino acids, more specifically, 7 to 18 or 9 to 16 amino acids, and much more specifically, it may include the amino acid sequence of MATTSTATTR (SEQ ID NO: 20).

In one specific embodiment, the single-chain insulin analog fused with a peptide may be that a peptide is fused to the N-terminus of a single-chain insulin analog in which native insulin A chain; the analog of the C-peptide; and native insulin B chain are linked in the above order, and specifically, it may have an amino acid sequence of SEQ ID NO: 19, but is not limited thereto.

The single-chain insulin analog according to the present invention encompasses any peptide having reduced insulin receptor binding affinity compared to native insulin, in which substitution, addition, deletion, or post-translational modification (for example, methylation, acylation, ubiquitination, intramolecular covalent bonds) of amino acids in the amino acid sequences of A chain, B chain, and C-peptide of native proinsulin is introduced. Upon substitution or addition of the amino acids, not only 20 amino acids commonly observed in human proteins, but also atypical or non-naturally occurring amino acids can be used. Commercial sources of atypical amino acids may include Sigma-Aldrich, ChemPep, and Genzyme Pharmaceuticals. Peptides including such amino acids and typical peptide sequences can be synthesized and purchased through commercial peptide synthesis companies such as American Peptide Company and Bachem of USA or Anygen of Korea.

More specifically, the single-chain insulin analog of the present invention may have Chemical Formula 2 below:

[Chemical Formula 2] X-Y-Z

wherein:
X is native insulin A chain, B chain, or an analog thereof,
Y is native insulin C-peptide or an analog thereof,
Z is native insulin B chain, A chain, or an analog thereof, and
with the proviso that cases where X, Y, and Z are B chain, C-peptide, and A chain of native insulin, respectively, or A chain, C-peptide, and B chain of native insulin, respectively, are excluded.

The insulin A chain, B chain, C-peptide, and analogs thereof are the same as described above.

The "-" refers to each bond between X, Y, and Z, and represents a direct bond or a linker between X, Y, and Z. The linker may be a peptide linker or a non-peptidyl linker.

In addition, the "-" may be any chemical bond such as a non-covalent bond, covalent bond, *etc.,* and specifically, it may be a covalent bond, but is not limited thereto.

Another aspect of the present invention provides a polynucleotide encoding the single-chain insulin analog, an expression vector comprising the polynucleotide, and a transformant comprising the expression vector.

The single-chain insulin analog is the same as described above.

The polynucleotide is deoxyribunucleotide (DNA) or ribonucleotide (RNA) existing in a single- or double-stranded form, and has a meaning including genomic DNA, cDNA, and RNA transcribed therefrom, and nucleotides, which are the basic structural units, include not only natural nucleotides but also analogs in which sugar or base sites are modified (Scheit, Nucleotide Analogs, John Wiley, New York, 1980*;* Uhlman and Peyman, Chemical Reviews, 90: 543-584, 1990). The polynucleotide of the present invention can be isolated or prepared using standard molecular biology techniques. For example, a suitable primer sequence can be used to amplify from a native proinsulin gene sequence (NM_000207.2, NCBI) via polymerase chain reaction (PCR), and can be prepared using the standard synthesis technique using an automated DNA synthesizer. The polynucleotide may be used interchangeably with nucleic acid.

The polynucleotide encoding the single-chain insulin analog may comprise the polynucleotides encoding the amino acid sequence of A chain, B chain, and C-peptide described above.

The recombinant vector according to the present invention can be typically constructed as vectors for cloning or vectors for expression, and as vectors for use of prokaryotic or eukaryotic cells as host cells.

As used herein, the term "vector" refers to a nucleic acid construct that is a recombinant vector capable of expressing a target protein in a suitable host cell and that includes essential regulatory elements operably linked to express a nucleic acid insert. The present invention can produce a recombinant vector comprising a nucleic acid encoding an insulin analog, and by transforming or transfecting the recombinant vector into a host cell, the single-chain insulin analog of the present invention can be obtained.

In the present invention, the nucleic acid encoding a single-chain insulin analog is operatively linked to a promoter. As used herein, the term "operatively linked" refers to a functional linkage between a nucleic acid expression control sequence (*e.g.:* a promoter, signal sequence, ribosomal binding site, transcription termination sequence, *etc*.) and other nucleic acid sequences, and the regulatory sequence thereby regulates transcription and/or translation of the other nucleic acid sequences.

As used herein, the term "promoter" refers to an untranslated nucleic acid sequence upstream of a coding region, comprising a binding site for a polymerase and having a transcription initiation activity to mRNA of a gene located downstream of a promoter, that is, a DNA region where the polymerase binds to initiate transcription of a gene, and is located at the 5' site of the mRNA transcription initiation site.

For example, when the vector of the present invention is a recombinant vector and the prokaryotic cell is a host cell, it is general to comprise a strong promoter to proceed with transcription (*e.g.:* tac promoter, lac promoter, lacUV5 promoter, lpp promoter, pLλ promoter, pRλ promoter, rac5 promoter, amp promoter, recA promoter, SP6 promoter, trp promoter, T7 promoter, *etc*.), a ribosome binding site for initiation of translation, and a transcription/translation terminating sequence.

In addition, vectors that can be used in the present invention can be produced by manipulating plasmids (*e.g.:* pSC101, pGV1106, pACYC177, ColE1, pKT230, pME290, pBR322, pUC8/9, pUC6, pBD9, pHC79, pIJ61, pLAFR1, pHV14, pGEX series, pET series, pPICZα series, pUC19, *etc*.), phages (e.g.: λgt4 • λB, λ-Charon, λΔz1, M13, *etc.*), or viruses (e.g.: SV40, *etc.*) that are often used in the art.

Meanwhile, when the vector of the present invention is a recombinant vector and the eukaryotic cell is a host cell, promoters derived from the genome of mammalian cells (*e.g.:* metallothionine promoters) or promoters derived from mammalian viruses (*e.g.:* adenovirus late promoters, vaccinia virus 7.5K promoters, SV40 promoters, cytomegalovirus promoters, and tk promoter of HSV) can be used, and it is general to have a polyadenylation sequence (*e.g.:* bovine growth hormone terminator and polyadenylation sequences derived from SV40).

In addition, the recombinant vector of the present invention comprises antibiotic resistant genes conventionally used in the art as selection markers, and for example, genes resistant to ampicillin, gentamicin, carbenicillin, chloramphenicol, streptomycin, kanamycin, geneticin, neomycin, and tetracycline can be used.

The recombinant vector of the present invention may further comprise other sequences as necessary to facilitate purification of the target protein to be recovered, namely, insulin analog. The sequences that can be included additionally may be protein tablet tag sequences, such as glutathione S-transferase (Pharmacia, USA), maltose binding protein (NEB, USA), FLAG (IBI, USA), and hexahistidine, *etc.,* but the examples do not limit the type of sequences required for purification of target proteins.

Fusion proteins expressed by recombinant vectors comprising such tag sequences can be purified by affinity chromatography. For example, when glutathione-S-transferase is fused, glutathione, which is a substrate of the enzyme, can be used, and when hexahistidine tags are used, target proteins can be easily recovered by using a Ni-NTA column.

Using a recombinant vector comprising a polynucleotide encoding the single-chain insulin analog, a transformant transformed with the vector can be constructed.

As used herein, the term "transformation" refers to a phenomenon, in which DNA is introduced into a host cell to allow DNA to be replicated as a factor of a chromosome or by completion of chromosome integration, and external DNA is introduced into cells to artificially cause genetic changes.

As the transformation method of the present invention, any transformation method may be used, and may be easily performed according to conventional methods in the art. In general, for the transformation method, there are the CaCl₂ precipitation method, the Hanahan method which improves efficiency by using a reducing material called dimethyl sulfoxide (DMSO) in the CaCl₂ precipitation method, electroporation, calcium phosphate precipitation method, plasma fusion method, agitation method with silicon carbide fibers, agrobacterial mediated transformation method, transformation method using PEG, dextran sulfate, lipofectamine, drying/inhibition mediated transformation method, *etc.*

The method for transforming a recombinant vector comprising a nucleic acid encoding a single-chain insulin analog according to the present invention is not limited to the above examples, and transformation or transfection methods conventionally used in the art may be used without limitation.

The transformant of the present invention can be obtained by introducing into a host cell a recombinant vector comprising a nucleic acid encoding a single-chain insulin analog, which is a target nucleic acid.

The hosts suitable for the present invention are not particularly limited as long as they allow the nucleic acid of the present invention to be expressed. Specific examples of the hosts that can be used in the present invention include bacteria of the genus *Escherichia,* such as *E. coli;* bacteria of the genus *Bacillus,* such as *Bacillus subtilis;* bacteria of the genus *Pseudomonas,* such as *Pseudomonas putida;* yeasts such as *Pichia pastoris, Saccharomyces cerevisiae,* and *Schizosaccharomyces pombe;* insect cells such as *Spodoptera frugiperda* (SF9); and animal cells such as CHO, COS, BSC, *etc.,* but are not limited thereto. Specifically, *E. coli* is used as a host cell.

Another aspect for implementing the present invention provides a method for preparing a single-chain insulin analog using the transformant.

Specifically, the present invention provides a method for preparing the single-chain insulin analog, comprising:
a) expressing a single-chain insulin analog by culturing a transformant comprising a nucleic acid encoding the single-chain insulin analog; and
b) isolating and purifying the expressed single-chain insulin analog.

The single-chain insulin analog, nucleic acid, and transformant are the same as described above.

The medium used for culturing the transformant in the present invention should meet the requirement of host cell culture in an appropriate manner. The carbon source that may be included in the medium for the growth of the host cell may be appropriately selected at the judgment of those skilled in the art according to the type of the prepared transformant, and appropriate culture conditions may be adopted to control the timing and amount of culture.

Sugar sources that can be used may include sugars and carbohydrates such as glucose, saccharose, lactose, fructose, maltose, starch, and cellulose; oils and fats such as soybean oil, sunflower oil, castor oil, and coconut oil; fatty acids such as palmitic acid, and stearic acid, linoleic acid; alcohols such as glycerol, ethanol; and organic acids such as acetic acid. These materials can be used alone or in combination.

Nitrogen sources that can be used may include peptone, yeast extract, gravy, malt extract, corn steep liquor, soybean wheat, and urea, or inorganic compounds such as ammonium sulfate, ammonium chloride, ammonium phosphate, ammonium carbonate, and ammonium nitrate. Nitrogen sources can also be used individually or as a mixture.

Phosphorous sources that can be used may include potassium dihydrogen phosphate or dipotassium hydrogen phosphate, or the corresponding sodium-containing salts thereof. In addition, the culture medium may contain a metal salt such as magnesium sulfate or iron sulfate required for growth.

Finally, in addition to these materials, essential growth materials such as amino acids and vitamins can be used. In addition, suitable precursors for culture media can be used. The above-mentioned raw materials can be added batchwise or continuously in a manner appropriate to the culture during the cultivation. Basic compounds such as sodium hydroxide, potassium hydroxide, and ammonia, or acidic compounds such as phosphoric acid or sulfuric acid can be used in an appropriate manner to adjust the pH of the culture. In addition, antifoaming agents, such as fatty acid polyglycol esters, can be used to inhibit bubble generation. In order to maintain aerobic conditions, oxygen or oxygen-containing gas (*e.g.:* air) is injected into the culture.

Culturing of the transformant according to the present invention is usually carried out at a temperature of 20°C to 45°C, specifically, 25°C to 40°C. In addition, the culture is continued until the maximum amount of the single-chain insulin analog desired is obtained, and for this purpose, the culture can usually last for 10 hours to 160 hours.

As described above, if the appropriate culture conditions are established depending on the host cell, the transformant according to the present invention will produce a single-chain insulin analog, and depending on the composition of the vector and the characteristics of the host cell, the single-chain insulin analog produced can be secreted into the cytoplasm of the host cell, into the periplasmic space, or extracellularly.

Proteins expressed in the host cell or outside thereof can be purified in a conventional manner. Examples of purification methods include salting out (*e.g.:* ammonium sulfate precipitation, sodium phosphate precipitation, *etc.*), solvent precipitation (*e.g.:* protein fractionation precipitation using acetone, ethanol, *etc*.), dialysis, gel filtration, ion exchange, chromatography such as reverse phase column chromatography, ultrafiltration, *etc.* can be used alone or in combination.

In a specific embodiment of the present invention, a method for preparing a single-chain insulin analog may comprise:
(a) expressing a single-chain insulin analog in a form, in which a peptide consisting of 5 to 20 amino acids comprising a protease cleavage site is fused to an N-terminus of the single-chain insulin analog; and
(b) removing the peptide fused to the single-chain insulin analog.

The peptide fused to the single-chain insulin N-terminus contributes to an increase in expression yield of the single-chain insulin analog, and has a protease (for example, trypsin) cleavage site, and is efficiently removed thereby upon production of single-chain insulin analogs. The peptide added to the single-chain insulin N-terminus is not limited as long as it contributes to an increase in expression yield of the single-chain insulin analog and it comprises a protease cleavage site. The peptide may consist of 5 to 20 amino acids, more specifically, 7 to 18, or 9 to 16 amino acids, much more specifically, may comprise an amino acid sequence of MATTSTATTR (SEQ ID NO: 20). The single-chain insulin analog fused with the peptide may have an amino acid sequence of SEQ ID NO: 24, but is not limited thereto.

In one specific exemplary embodiment, the single-chain insulin analog fused with a peptide may be that a peptide is fused at the N-terminus of a single-chain insulin analog in which native insulin A chain; the analog of the C-peptide; and native insulin B chain are connected in the above order, and specifically, may have an amino acid sequence of SEQ ID NO: 19, but is not limited thereto.

In an embodiment of the present invention, the following steps may be further included to isolate and purify the single-chain insulin analog expressed in the form of an inclusion body from a transformant.
b-1) harvesting and crushing a transformant from the culture medium of step a);
b-2) recovering and refolding a single-chain insulin analog expressed in a crushed cell lysate;
b-3) purifying the refolded single-chain insulin analog by cation exchange chromatography;
b-4) treating the purified single-chain insulin analog with a protease (for example, trypsin or carboxypeptidase B); and/or
b-5) purifying the treated single-chain insulin analog by cation exchange chromatography, anion exchange chromatography, reverse phase chromatography, or a combination thereof.

In addition, the isolating and purifying step may further comprise removing the peptide fused to the single-chain insulin analog, when the single-chain insulin analog is expressed in form where the peptide comprising a protease cleavage site is fused to the single-chain insulin analog.

Still another aspect of the present invention provides a formulation that can increase the half-life and bioavailability of single-chain insulin analogs, or maintain activity continuously. Specifically, the formulation refers to a formulation comprising a carrier directly covalently bonded to a single-chain insulin analog, or a formulation comprising a component capable of increasing the *in vivo* activity of the single-chain insulin analog even in the absence of a direct covalent bond.

In addition, still another aspect of the present invention provides a single-chain insulin analog conjugate as a long-acting insulin, in which a single-chain insulin analog and a material that can increase its *in vivo* half-life are linked together. In addition, the single-chain insulin analog of the present invention has a lower activity than native insulin, thereby lowering the risk of hypoglycemia, which is the biggest problem of existing native insulin. This long-acting formulation can maintain a low activity and is thus advantageous for a long-term control of blood glucose levels without the risk of hypoglycemia.

In one specific embodiment, the single-chain insulin analog conjugate of the present invention has a structure of Chemical Formula 1 below:

[Chemical Formula 1] X-Y-Z-La-F

wherein:
X is native insulin A chain, B chain, or an analog thereof,
Y is native insulin C-peptide or an analog thereof,
Z is native insulin B chain, A chain, or an analog thereof,
L is a linker,
A is 0 or a natural number, with the proviso that when a is 2 or more, each L is independent of each other,
F is a material capable of increasing an *in vivo* half-life of an insulin analog,
with the proviso that cases where X, Y, and Z are a B chain, C-peptide, and A chain of native insulin, respectively, or an A chain, C-peptide, and B chain of native insulin, respectively, are excluded, and
X-Y-Z forms a single-chain insulin analog.

The "-" means each bond, and indicates a direct bond or a linker. The linker may be a peptidyl linker or a nonpeptidyl linker.

In addition, "-" may be any chemical bond such as a non-covalent bond or a covalent bond, and specifically, may be a covalent bond, but is not limited thereto.

The L may be a peptidyl polymer or a nonpeptidyl polymer.

When the L is a peptidyl polymer, it may comprise one or more amino acids, for example, may include 1 to 1,000 amino acids, but is not particularly limited thereto. Various known peptidyl linkers may be used in the present invention to link F and Z in the present invention, and comprise, for example [GS]ₓ linker, [GGGS]ₓ linker, and [GGGGS]ₓ linker, *etc.,* and here, x may be a natural number of 1 or more. However, it is not limited to the above embodiments.

When L is a nonpeptidyl polymer, the nonpeptidyl polymer may be selected from the group consisting of polyethylene glycol, polypropylene glycol, ethylene glycol-propylene glycol copolymer, polyoxyethylated polyol, polyvinyl alcohol, polysaccharide, dextran, polyvinyl ethyl ether, biodegradable polymer, lipid polymer, chitin, hyaluronic acid, and a combination thereof, and more specifically, it may be polyethylene glycol, but is not limited thereto.

The X-Y-Z and F may be bonded to each other by a covalent or non-covalent chemical bond, and X-Y-Z and F may be bonded to each other through L via a covalent chemical bond, a non-covalent chemical bond, or a combination thereof.

As used herein, the term "long-acting insulin" refers to a material in which a single-chain insulin analog is combined with a biocompatible material capable of extending the half-life. The long-acting insulin has an increased half-life compared to native insulin.

The single-chain insulin analog is the same as described above.

In the present invention, "biocompatible material or material capable of increasing t*in vivo* half-life" refers to a material, which can be linked to a single-chain insulin analog and extend its half-life. "The biocompatible material" or "material capable of increasing *in vivo* half-life" is used interchangeably with "carrier" in the present invention.

The biocompatible material or carrier comprise any material that can be linked to a single-chain insulin analog and extend its half-life, and for example, may be selected from the group consisting of polyethylene glycol, fatty acid, cholesterol, albumin and a fragment thereof, albumin binding agent, polymers of repeating units of specific amino acids, antibody, antibody fragment, FcRn binding material, *in vivo* connective tissue or derivative thereof, nucleotide, fibronectin, transferrin, saccharide, and polymer, but is not limited thereto.

The biocompatible material or carrier may be linked to a single-chain insulin analog by a covalent or non-covalent bond. In addition, the linkage of the single-chain insulin analog with the biocompatible material or carrier includes genetic recombination methods and an *in vitro* binding using polymers, low molecular weight chemicals, *etc.,* but is not limited to any of the binding methods.

In the present invention, when using polyethylene glycol as a carrier, it may include Ambrx's Recode technique that can attach polyethylene glycol in a site-specific manner, and may include Neose's glycopegylation technique that may specifically attach polyethylene glycol to sugar chain sites. In addition, it may include a releasable PEG technique in which polyethylene glycol is slowly removed *in vivo,* but is not limited thereto, and it may include a technique for enhancing bioavailability using PEG.

In addition, polymers such as polyethylene glycol, polypropylene glycol, ethylene glycol-propylene glycol copolymer, polyoxyethylated polyol, polyvinyl alcohol, polysaccharide, dextran, polyvinyl ethyl ether, biodegradable polymer, lipid polymer, chitin, and hyaluronic acid can be linked to an insulin analog using the above techniques.

In the present invention, when albumin is used as a carrier, the following techniques may be included: a techniquein which albumin or albumin fragments can be directly covalently bound to an insulin analog to increase *in vivo* stability; a technique in which an albumin-binding material (*e.g.,* albumin-specific binding antibodies or antibody fragments) can be linked to an insulin analog thereby allowing the insulin analog to be bound to albumin, although albumin itself is not directly bound to the insulin analog, and a technique in which specific peptides/proteins having a binding affinity to albumin (*e.g.,* albumin binding peptides produced using Affibody's albumod technique) can be linked to an insulin analog; and a technique in which fatty acids, *etc.* having a binding affinity to albumin can be linked to an insulin analog, but the techniques to be used are not limited thereto, and any technique, binding method, *etc.,* which can increase *in vivo* stability using albumin can be included.

A technique for linking to an insulin analog using an antibody or antibody fragment as a carrier to increase *in vivo* half-life may also be included in the present invention. It may be an antibody or antibody fragment having an FcRn binding site, and may be any antibody fragment that does not include an FcRn binding site such as Fab, *etc.* The CovX-body technique using catalytic antibodies by CovX may be included therein, and a technique of increasing *in vivo* half-life using an immunoglobulin Fc region may be included in the present invention.

The FcRn binding material may be an immunoglobulin Fc region.

As used herein, the term "immunoglobulin Fc region" refers to the rest of an immunoglobulin excluding the heavy and light chain variable regions, heavy chain constant region 1 (CHI), and light chain constant region (CL), and the immunoglobulin Fc region may further comprise a hinge region in the heavy chain constant region. In particular, it may be a fragment including a part or the entirety of the immunoglobulin Fc region, so that the immunoglobulin Fc region can be used interchangeably with an immunoglobulin fragment or an immunoglobulin constant region.

While native Fc has a sugar chain at the Asn297 site in the heavy chain constant region 1, an *E*. *coli*-derived recombinant Fc is expressed in a form without a sugar chain. When the sugar chain is removed from an Fc, the binding affinity of the Fc gamma receptors 1, 2, 3 and complement (c1q) that bind to the heavy chain constant region 1 is reduced, thereby reducing or eliminating antibody-dependent cytotoxicity or complement-dependent cytotoxicity.

As used herein, the term "immunoglobulin constant region" refers to an Fc fragment including a heavy chain constant region 2 (CH2) and a heavy chain constant region 3 (CH3) (or including a heavy chain constant region 4 (CH4)) portions, excluding the heavy and light chain variable regions, heavy chain constant region 1 (CH1), and light chain constant region (CL) of an immunoglobulin, and it may comprise a hinge region in the heavy chain constant region. In addition, as long as the immunoglobulin constant region of the present invention has a substantially equivalent or improved effect compared to the native form, it may be an extended immunoglobulin constant region comprising a part or the entirety of the heavy chain constant region 1 (CH1) and/or light chain constant region (CL), excluding the heavy and light chain variable regions of the immunoglobulin. In addition, the immunoglobulin constant region may also be a region from which a fairly long amino acid sequence corresponding to CH2 and/or CH3 has been removed. That is, the immunoglobulin constant region of the present invention may be (1) CH1 domain, CH2 domain, CH3 domain, and CH4 domain, (2) CH1 domain and CH2 domain, (3) CH1 domain and CH3 domain, (4) CH2 domain and CH3 domain, (5) a combination of one or two or more constant region domains and an immunoglobulin hinge region (or a part of the hinge region), and (6) a dimer of each domain of a heavy chain constant region and a light chain constant region. Constant regions, including immunoglobulin Fc fragments, are safe for use as carriers of drugs because they are biodegradable polypeptides that are metabolized *in vivo.* In addition, immunoglobulin Fc fragments are advantageous in terms of preparation, purification, and yield of conjugates, because they have a relatively low molecular weight compared to the entire immunoglobulin molecule. Additionally, since the amino acid sequence varies from antibody to antibody, the Fab moiety exhibiting high non-homogeneity is removed, so that effects can be expected where the homogeneity of materials is greatly increased, and the possibility of inducing blood antigens is also lowered.

Meanwhile, the immunoglobulin constant region may be of human or animal origin such as cattle, goats, pigs, mice, rabbits, hamsters, rats, guinea pigs, *etc.,* and specifically, is of human origin. In addition, the immunoglobulin constant region may be selected from the group consisting of constant regions derived from IgG, IgA, IgD, IgE, and IgM or a combination thereof, or a hybrid thereof. Specifically, the immunoglobulin constant region may be derived from IgG or IgM, which is most abundant in human blood, and most specifically, from IgG known to enhance the half-life of ligand-binding proteins. In the present invention, the immunoglobulin Fc region may be a dimer or multimer consisting of single-chain immunoglobulins consisting of domains of the same origin.

As used herein, the term "combination" means that when forming a dimer or multimer, a polypeptide encoding the single-chain immunoglobulin constant region of the same origin (specifically an Fc region) forms a bond with a single-chain polypeptide of different origin. That is, the preparation of a dimer or multimer is possible from two or more fragments selected from the group consisting of the fragments of IgG Fc, IgAFc, IgM Fc, IgD Fc, and IgE.

As used herein, the term "hybrid" means that a sequence corresponding to two or more immunoglobulin constant regions of different origins exists in a single-chain immunoglobulin constant region (specifically, an Fc region). In the case of the present invention, various types of hybrids are possible. That is, hybrids of domains composed of 1 to 4 domains selected from the group consisting of CH1, CH2, CH3, and CH4 of IgG Fc, IgM Fc, IgAFc, IgE Fc, and IgD Fc are possible, and may further comprise a hinge region.

IgG can also be divided into subclasses of IgG1, IgG2, IgG3, and IgG4, and combinations or hybridized forms thereof are also possible in the present invention. Specifically, IgG may be an IgG2 and IgG4 subclass, and more specifically, an Fc region of IgG4 having little effector function such as complement dependent cytotoxicity (CDC).

In addition, the immunoglobulin constant region can be in the form of a native sugar chain, an increased sugar chain compared to the native form, a reduced sugar chain compared to the native form, or with a sugar chain removed. Conventional methods such as chemical methods, enzymatic methods, and genetic engineering using microorganisms can be used to increase or decrease the sugar chains of the immunoglobulin constant region. In this case, in the immunoglobulin constant region from which a sugar chain is removed from the immunoglobulin constant region, the binding affinity of the complement (c1q) is significantly reduced. In particular, since the antibody-dependent cytotoxicity or complement-dependent cytotoxicity is reduced or eliminated, the immunoglobulin constant region does not cause an unnecessary immune response *in vivo.* In this regard, a form more consistent with its original purpose as a drug carrier would be an immunoglobulin constant region in which sugar chains are removed or aglycosylated. Therefore, much more specifically, aglycosylated Fc regions derived human, that is, human aglycosylated IgG4Fc regions can be used. Human-derived Fc regions may be preferred over non-human derived Fc regions that can cause undesirable immune responses, such as acting as antigens in the human body and generating new antibodies against them.

In addition, the immunoglobulin constant region of the present invention includes native amino acid sequences as well as the sequence derivatives thereof (mutants). An amino acid sequence derivative means that one or more amino acid residues in the native amino acid sequence have different sequences by deletion, insertion, non-conservative or conservative substitution, or a combination thereof. For example, in the case of IgG Fc, the 214^{th} to the 238^{th}, the 297^{th} to the 299^{th}, and the 318^{th} to the 322^{nd}, or the 327^{th} to the 331^{st} amino acid residues that are known to be important for binding can be used as suitable sites for modification. In addition, various types of derivatives are possible, where sites that can form disulfide bonds are removed, some amino acids at the N-terminus in native Fc are removed, or methionine residues are added to the N-terminus of native Fc, *etc.* In addition, complement-binding sites, such as C1q binding sites, may be removed, or ADCC sites may be removed to eliminate the effector function. Techniques for preparing sequence derivatives of such immunoglobulin constant regions are disclosed in WO 97/34631, WO 96/32478, *etc.*

Amino acid exchange in proteins and peptides that do not alter the activity of molecules as a whole are known in the art (H. Neurath, R.L. Hill, The Proteins, Academic Press, New York, 1979). Exchange that conventionally occurs the most is exchange between amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly. Depending on cases, it may be modified by phosphorylation, sulfation, acrylation, glycosylation, methylation, farnesylation, acetylation, amidation, *etc.*

While the immunoglobulin constant region derivative described above exhibits the same biological activity as the immunoglobulin constant region of the present invention, it may be a derivative where structural stability is enhanced against heat, pH, *etc.* of the immunoglobulin constant region. In addition, such immunoglobulin constant regions may be obtained from native forms isolated *in vivo* from humans and animals such as cattle, goats, pigs, mice rabbits, hamsters, rats, guinea pigs, *etc.,* and these may be a recombinant type or a derivative thereof obtained from a transformed animal cell or microorganism. In this case, the method of obtaining from the native form is to obtain by isolating the whole immunoglobulin from living bodies of human or animal, and then treating with protease. When treated with papain, the whole immunoglobulin is cleaved to Fab and Fc, and when treated with pepsin, the whole immunoglobulin is cleaved to pF'c and F(ab)2. Fc or pF'c can be separated from these fragments by using size-exclusion chromatography, *etc.*

Specifically, the human-derived immunoglobulin constant region may be a recombinant immunoglobulin constant region obtained from a microorganism.

In one specific embodiment, in the conjugate of the present invention, a single-chain insulin analog and a material capable of increasing the *in vivo* half-life thereof may be linked via a linker.

In the present invention, the linker may be in a form where the linker is linked to the N-terminus, C-terminus, lysine, histidine, or cysteine of each of a single-chain insulin analog or a material capable of increasing the *in vivo* half-life thereof.

The linker may be a peptidyl linker or nonpeptidyl linker.

As the nonpeptidyl linker, a protease-resistant polymer can be used to maintain the blood half-life of a single-chain insulin analog, similar to a material capable of increasing the *in vivo* half-life of the single-chain insulin analog. Therefore, the nonpeptidyl linker that can be used in the present invention can be used without limitation as long as it is a nonpeptidyl polymer having the above-mentioned role, that is, having resistance to proteases *in vivo.*

As used herein, the term "nonpeptidyl polymer" includes a biocompatible polymer having two or more repeating units bound thereto, and is used interchangeably with the term "nonpeptidyl linker." The repeating units are bound to each other through any covalent bond, not a peptide bond. In the present invention, the nonpeptidyl polymer may include a reactor at its end to form a conjugate through a reaction with other components constituting the conjugate.

As used herein, the term "nonpeptide linkage moiety" refers to a component in a conjugate formed by binding a nonpeptidyl polymer having a reactive group at both ends with an immunoglobulin Fc region and a single-chain insulin analog through each reactive group.

In one specific embodiment, the single-chain insulin analog conjugate may be that the immunoglobulin Fc region and the single-chain insulin analog are covalently bound to each other, through a nonpeptidyl polymer comprising a reactive group capable of linking an immunoglobulin Fc region and a single-chin insulin analog at both ends of the nonpeptidyl polymer.

Specifically, while not particularly limited hereto, the nonpeptidyl polymer may be selected from the group consisting of polyethylene glycol, polypropylene glycol, copolymers of ethylene glycol and propylene glycol, polyoxy ethylated polyols, polyvinyl alcohol, polysaccharide, dextran, polyvinyl ethyl ether, biodegradable polymers such as polylactic acid (PLA) and polylactic-glycolic acid (PLGA), lipid polymers, chitin, hyaluronic acid, oligonucleotides, and a combination thereof. In more specific embodiment, the nonpeptidyl polymer may be polyethylene glycol, but is not limited thereto. In addition, those derivatives already known in the art and derivatives that can be easily prepared at the technical level in the art are included in the scope of the present invention.

The linkage by the linker may be any chemical bond such as a non-covalent chemical bond or a covalent chemical bond, but is not limited thereto.

More specifically, in the present invention, the nonpeptidyl polymer includes a biocompatible polymer having two or more repeating units linked thereto, and the repeating units are bound to each other through any covalent bond, not a peptide bond. Such nonpeptidyl polymers may have two ends or three ends.

The nonpeptidyl polymer that can be used in the present invention may be used without limitation as long as it is a polymer having resistance to proteases *in vivo,* and specifically, the molecular weight of the nonpeptidyl polymer may be in a range of over 0 kDa to 200 kDa, specifically, a range of 1 kDa to 100 kDa, more specifically, a range of 1 kDa to 50 kDa, much more specifically, a range of 1 kDa to 20 kDa, much more specifically, 3.4 kDa to 10 kDa, and much more specifically, about 3.4 kDa, but is not limited thereto.

In addition, for the carrier, in particular, the nonpeptidyl polymer of the present invention linked to an immunoglobulin Fc region, not only one type of polymer but also a combination of different types of polymers may be used.

In one specific exemplary embodiment, both ends of the nonpeptidyl polymer may each bind to an amine group, thiol group, or hydroxy group of an immunoglobulin Fc region or a single-chain insulin analog.

Specifically, the nonpeptidyl polymer may comprise a reactive group capable of linking each of immunoglobulin Fc and a single-chain insulin analog at both ends, specifically, a reactive group capable of linking amine groups located at the N-terminus, lysine, and/or histidine, hydroxy groups located at the C-terminus and/or thiol groups of cysteine of the single-chain insulin analog or immunoglobulin Fc region, but is not limited thereto.

More specifically, the reactive group of the nonpeptidyl polymer may be one or more selected from the group consisting of an aldehyde group, propionaldehyde group, butyl aldehyde group, maleimide group, and succinimide derivative, but is not limited thereto.

In the above, examples of the aldehyde group include, but are not limited to, a propionaldehyde group or a butyl aldehyde group, but is not limited thereto.

In the above, for succinimide derivatives, succinimidyl carboxymethyl, succinimidyl valerate, succinimidyl methylbutanoate, succinimidyl methylpropionate, succinimidyl butanoate, succinimidyl propionate, N-hydroxysuccinimide phosphorus or succinimidyl carbonate may be used, but is not limited thereto.

The nonpeptidyl polymer may be linked to an immunoglobulin Fc and a single-chain insulin analog via the reactive group as above and converted to a nonpeptidyl polymer linkage moiety.

In addition, the final product produced by reductive alkylation by aldehyde bonds is much more stable than when linked by amide bonds. The aldehyde reactive group selectively reacts at the N-terminus at low pH and can form covalent bonds with lysine residues at high pH, for example, pH 9.0 conditions.

The reactive end group of the nonpeptidyl polymer of the present invention may be the same or different from each other. The nonpeptidyl polymer may have an aldehyde group reactive group at the end, and the nonpeptidyl polymer may have each of an aldehyde group and maleimide reactive group at the end, or may have each of an aldehyde group or succinimide group at the end, bus is not limited thereto.

For example, the nonpeptidyl polymer may have a maleimide group at one end, and an aldehyde group, a propionaldehyde group, or a butyraldehyde group at the other end. As another example, it may have a succinimidyl group at one end and a propionaldehyde group or a butyraldehyde group at the other end.

When poly(ethylene glycol) having a hydroxy reactive group at the propion-side end is used as a nonpeptidyl polymer, the conjugate of the present invention can be prepared using known chemical reactions to activate the hydroxy group into the various reactive groups, or by using poly(ethylene glycol) having a modified reactive group that is commercially available.

In one specific embodiment, the reactive group of the nonpeptidyl polymer may be linked to the cysteine residue of a single-chain insulin analog, more specifically, to the -SH group of cysteine, but is not limited thereto.

If maleimide-PEG-aldehyde is used, the maleimide group can be linked to the -SH group of a single-chain insulin analog by a thioether bond, and the aldehyde group can be linked to the - NH₂ group of an immunoglobulin Fc through a reductive alkylation reaction, but the linkage is not limited thereto, and this is merely an embodiment.

Through such reductive alkylation, the N-terminal amino group of the immunoglobulin Fc region and an oxygen atom located at one end of PEG are linked to each other via a linker reactive group having a structure of -CH₂CH₂CH₂- to form a structure such as -PEG-O-CH₂CH₂CH₂NH-immunoglobulin Fc, and a thioether bond can form a structure in which one end of PEG is connected to a sulfur atom located in the cysteine of a single-chain insulin analog. The thioether bond described above may include the structure of

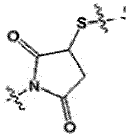

However, the linkage is not particularly limited to the above-mentioned example, and this is merely an embodiment.

In addition, in the conjugate, the reactive group of a nonpeptidyl polymer may be linked to -NH₂ located at the N-terminus of the immunoglobulin Fc region, but this is merely an embodiment. Specifically, the single-chain insulin analog of the present invention may be linked via the C-terminus with a nonpeptidyl polymer having a reactive group.

In the present invention, "C-terminus" refers to the carboxy terminus of a peptide, and refers to a position capable of binding to a nonpeptidyl polymer for the purpose of the present invention. For example, while not particularly limited hereto, C-terminus may include all of the amino acid residues near the C-terminus as well as the most terminal amino acid residues of the C-terminus, and specifically, it may include the 1^{st} to 20^{th} amino acid residues from the most terminal.

In one specific embodiment, the linkage of a single-chain insulin analog to a material capable of extending *in vivo* half-life may be performed by a genetic recombination method.

Another aspect of the present invention provides a method for preparing a single-chain insulin analog, comprising linking the single-chain insulin analog to a material capable of increasing an *in vivo* half-life thereof.

The single-chain insulin analog, a material capable of increasing an *in vivo* half-life thereof, and the single-chain insulin analog conjugate are the same as described above.

Specifically, the method may comprise:
(a) preparing a linking moiety by reacting a nonpeptidyl polymer having two or more reactive end groups and one of a single-chain insulin analog or carrier, to attach the single-insulin analog or carrier to one end of the nonpeptidyl polymer, and to have a reactive end group at the other end thereof; and
(b) preparing a conjugate by reacting the linking moiety prepared in the above step (a) and the other one among a carrier or a single-chain insulin analog that is not attached to the connective body such that a single-chain insulin analog and a carrier are linked via a nonpeptidyl polymer.

The above-mentioned descriptions apply to the nonpeptidyl polymer, carrier, single-chain insulin analog, and their linkage constitutions.

As used herein, the term "linking moiety" refers to an intermediate body in which the nonpeptidyl polymer and only one of the single-chain insulin analog or carrier is covalently linked, and in the linking moiety, a single-chain insulin analog or carrier not attached to the linker may be attached to the end of the nonpeptidyl polymer, which is not linked to the single-chain insulin analog or carrier.

The single-chain insulin analog can be obtained according to the method for preparing the single-chain insulin analog described above, and can be prepared by commercial request.

Another aspect of the present invention provides a long-acting single-chain insulin formulation with enhanced *in vivo* duration and stability, comprising the single-chain insulin analog conjugate or the single-chain insulin analog.

Meanwhile, for formulations that can increase bioavailability or maintain persistent activity, sustained-release formulations prepared by using microparticles using PLGA, hyaluronic acid, chitosan, *etc.,* nanoparticles, *etc.* may be included therein.

In addition, for formulations of other forms that can increase bioavailability or maintain persistent activity, there may be implants, inhalation, nasal formulations, and formulations in the form of patches.

These single-chain insulin analogs or single-chain insulin analog conjugates of the present invention not only maintain the *in vivo* activity of existing insulin such as energy metabolism and sugar metabolism, but also significantly increase the blood half-life of insulin analogs and the *in vivo* activity persisting effect of the peptide thereby, and thus it is useful for the treatment of diabetes.

Another aspect of the present invention provides a pharmaceutical composition for preventing or treating diabetes, comprising the single-chain insulin analog or the single-chain insulin analog conjugate.

The single-chain insulin analog and the single-chain insulin analog conjugate are the same as described above.

As used herein, the term "prevention" refers to any activity that inhibits or delays the onset of diabetes by administration of the pharmaceutical composition, and the term "treatment" refers to any activity that improves or ameliorates the symptoms of diabetes by administration of the pharmaceutical composition.

As used herein, the term "administration" means introducing a certain material into a patient in any suitable manner, and the administration route of the pharmaceutical composition is not particularly limited, but the composition may be administered via any general route that can reach *in vivo* targets, for example, intraperitoneal administration, intravenous administration, intramuscular administration, subcutaneous administration, intradermal administration, oral administration, topical administration, nasal administration, intrapulmonary administration, or intrarectal administration, *etc.*

The pharmaceutical composition of the present invention may further comprise a pharmaceutically acceptable carrier, excipient, or diluent. Such pharmaceutically acceptable carriers, excipients, or diluents may be unnaturally occurring. The carrier is not particularly limited, but for oral administration, binders, lubricants, disintegrants, excipients, solubilizers, dispersants, stabilizers, suspending agents, colorants, fragrances, *etc.* may be used. In the case of injections, buffers, preservatives, analgesic agents, solubilizers, isotonic agents, stabilizers, *etc.* may be used in combination. For topical administration, bases, excipients, lubricants, preservatives, *etc.* may be used.

As used herein, the term "pharmaceutically acceptable" means that it is a sufficient amount to produce a therapeutic effect and does not cause a side effect, and it can be easily determined by those skilled in the art according to factors well known in the medical field such as the type of disease, age, weight, health, and gender of patients, sensitivity of patients to the drug, route of administration, method of administration, frequency of administration, duration of treatment, combination or concurrently used drugs, *etc.*

Formulation of the composition of the present invention can be prepared in various combinations with the pharmaceutically acceptable carrier described above. For example, for oral administration, it may be prepared in the form of tablets, troches, capsules, elixirs, suspensions, syrups, wafers, *etc.,* and in the case of injections, it may be prepared in unit dosage ampoules or in multiple dosage forms. It may be formulated into solutions, suspensions, tablets, pills, capsules, sustained release preparations, *etc.*

Meanwhile, for examples of carriers, excipients, and diluents suitable for formulation, lactose, dextrose, sucrose, sorbitol, mannitol, xylitol, erythritol, maltitol, starch, acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, *etc.* may be used. In addition, fillers, anticoagulants, lubricants, wetting agents, fragrances, preservatives, *etc.* may be further included.

In addition, the pharmaceutical composition of the present invention may have any one formulation selected from the group consisting of tablets, pills, powders, granules, capsules, suspensions, liquid solutions for internal use, emulsions, syrups, sterile aqueous solutions, nonaqueous solvents, lyophilized preparations, and suppositories.

In addition, the composition is formulated in a unit dosage form suitable for intrabody administration of a patient according to conventional methods in the pharmaceutical field, specifically, formulated into a form useful for the administration of protein drugs, and can be administered by using the administration method conventionally used in the art, such as oral or parenteral administration routes including skin, intravenous, intramuscular, intra-arterial, intramedullary, intrathecal, intraventricular, lung, transdermal, subcutaneous, intraperitoneal, intranasal, digestive tract, topical, sublingual, intravaginal or rectal routes, but is not limited thereto.

In addition, the conjugate may be used by mixing with various carriers accepted as a drug such as physiological saline or organic solvents, and in order to increase stability or absorption, carbohydrates such as glucose, sucrose, or dextran; antioxidants such as ascorbic acid or glutathione; other stabilizers such as chelating agents, low molecular weight proteins, *etc.* may be used as drugs.

The dosage and frequency of the pharmaceutical composition of the present invention is determined according to the type of drug as the active ingredient, together with various related factors such as the disease to be treated, route of administration, the age, gender, and body weight of the patient, the severity of disease, *etc.*

The total effective amount of the composition of the present invention may be administered to a patient in a single dose and may be administered by a fractionated treatment protocol that is administered in multiple doses for a long period of time. The pharmaceutical composition of the present invention may have a varied content of the active ingredient depending on the severity of the disease. Specifically, the preferred total dose of the conjugate of the present invention may be about 0.0001 mg to 500 mg per kg of patient weight per day. However, since the dose of the conjugate for an effective dose for a patient is determined considering the route of administration and frequency of treatment of the pharmaceutical composition, as well as various factors such as the age, weight, health status, and gender of the patient, severity of the disease, diet, excretion rate, *etc.,* those skilled in the art would be able to determine the appropriate effective dosage for the particular use of the composition of the present invention. The pharmaceutical composition according to the present invention is not particularly limited with regard to its formulation, administration route, and administration method, as long as the effect of the present invention is shown.

Still another aspect of the present invention provides a method for preventing or treating diabetes, comprising administering a pharmaceutical composition comprising the single-chain insulin analog or single-chain insulin analog conjugate to a subject in need thereof.

The single-chain insulin analog, single-chain insulin analog conjugate, prevention, and treatment are the same as described above.

As used herein, the term "subject" is a subject suspected of having diabetic disease, and the subject suspected of having diabetic disease refers to a mammal including a mouse, a livestock, *etc.* including a human who has the disease or may develop the disease. Additionally, a subject that can be treated with a single-chain insulin analog, single-chain insulin analog conjugate, or the composition comprising the same is included without limitation.

The method of the present invention can administer the pharmaceutical composition in a pharmaceutically effective amount. Appropriate total daily dose may be determined by the physician within the scope of good medical judgment and may be administered once or in multiple divided doses. However, for the purposes of the present invention, the specific therapeutically effective amount is preferably applied differently based on various factors and similar factors well known in the medical field such as the type and severity of the response to be achieved and whether other agents are used in some cases, as well as the specific composition, age, weight, general health, gender, and diet of the patient, time of administration, route of administration, the secretion rate of the composition, duration of treatment, the drug used in combination or concurrently used with the specific composition.

Hereinafter, the present invention will be described in more detail with reference to the examples. These examples are only for illustrating the present invention more specifically, but the scope of the present invention is not limited by these examples.

### Example 1: Preparation of single-chain insulin analog expressing vector

### 1. Preparation of proinsulin analog (A-chain-C peptide-B chain)

An expression vector with a nucleotide sequence was prepared to allow the expression of insulin analogs and proinsulin analogs in *E. coli.* The expression vector, to free the N-terminus of insulin A chain, comprised nucleotide sequences capable of expressing native proinsulin (B chain-C peptide-A chain; SEQ ID NOs: 4 and 5) and other proinsulin analog (A chain-C peptide-B chain, SEQ ID NOs: 6 and 7), and was prepared as below.

**[Table 1]**

| Analog | Sequence | | SEQ ID NO |
|---|---|---|---|
| Native proinsulin (B-C-A) | DNA | TTT GTG AAC CAA CAC CTG TGC GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC TAG | 4 |
| | Protein | FVNQHLCGSHLVEALYLVCGERGFFYTPKTRREAED LQVGQVELGGGPGAGSLQPLALEGSLQKRGIVEQCC TSICSLYQLENYCN | 5 |
| Single-chain insulin analog | DNA | GGC ATT GTG GAA CAA TGC TGT ACC AGC ATC TGC TCC CTC TAC CAG CTG GAG AAC TAC TGC AAC CGC CGG GAG GCA GAG GAC CTG CAG GTG | 6 |
| (A-C-B) | | GGG CAG GTG GAG CTG GGC GGG GGC CCT GGT GCA GGC AGC CTG CAG CCC TTG GCC CTG GAG GGG TCC CTG CAG AAG CGT TTT GTG AAC CAA CAC CTG TGC GGC TCA CAC CTG GTG GAA GCT CTC TAC CTA GTG TGC GGG GAA CGA GGC TTC TTC TAC ACA CCC AAG ACC TAG | |
| | Protein | GIVEQCCTSICSLYQLENYCNRREAEDLQVGQVELG GGPGAGSLQPLALEGSLQKRFVNQHLCGSHLVEALY LVCGERGFFYTPKT | 7 |

In order to prepare the expression vector of a proinsulin analog encoding 86 amino acids, 6 primers were synthesized based on the reported proinsulin gene sequence (NM_000207.2, NCBI). In addition, with proinsulin cDNA (Origene) as a template, A chain, C peptide, and B chain were amplified by PCR method, respectively, and then PCR was performed by mixing the 3 PCR amplification products with the primers to synthesize a proinsulin analog gene.

Specifically, for the synthesis of A chain of the proinsulin analog gene, a forward primer (SEQ ID NO: 8) was synthesized to comprise the initiation ATG codon and *Ndel* restriction enzyme site, and a reverse primer (SEQ ID NO: 9) was synthesized to comprise the 3' terminal region sequence of the A chain and the 5' region sequence of the C peptide. In order to synthesize a gene encoding C-peptide, a forward primer (SEQ ID NO: 10) was synthesized to comprise the N-terminal sequence of A chain and the sequence of C peptide, and a reverse primer (SEQ ID NO: 11) was synthesized to comprise the 3' terminal sequence of C peptide and 5' terminal sequence of B chain. In order to synthesize a gene encoding B chain, a forward primer (SEQ ID NO: 12) was synthesized to comprise the 3' terminal sequence of C peptide and the sequence of B chain, and a reverse primer (SEQ ID NO: 13) was synthesized to comprise the 5' terminal sequence of B chain and restriction enzyme *Ba*mHI sequence. The sequences of the above primers are as below.
5' GGAATTCCATATGGGCATTGTGGAACAATGCTGT 3' (SEQ ID NO: 8)
5' CTGCCTCCCGGCGGTTGCAGTAGTTCTCCAGCTG 3' (SEQ ID NO: 9)
5' GAACTACTGCAACCGCCGGGAGGCAGAGGACCTG 3' (SEQ ID NO: 10)
5' GTTGGTTCACAAAACGCTTCTGCAGGGACCCCTC 3' (SEQ ID NO: 11)
5' CCTGCAGAAGCGTTTTGTGAACCAACACCTGTGC 3' (SEQ ID NO: 12)
5' CGCGGATCCCTAGGTCTTGGGTGTGTAGAAGAAG 3' (SEQ ID NO: 13)

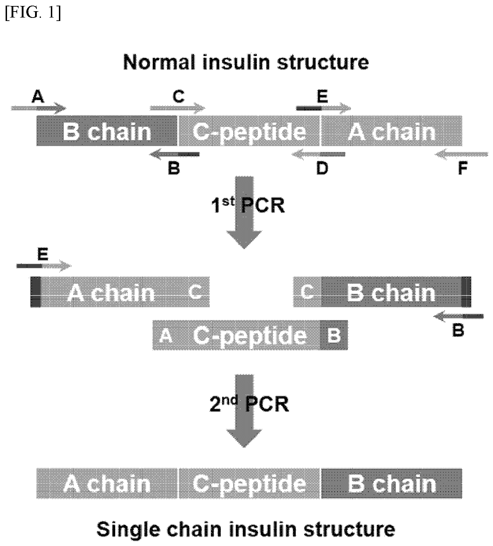

After amplifying each gene using the above primers, the PCR amplification products of each, an oligonucleotide represented by SEQ ID NO: 8, and a primer represented by SEQ ID NO: 13 were mixed together to synthesize a proinsulin analog having the final A chain-C peptide-B chain sequence (FIG. 1). For the amplification of the proinsulin analog, PCR was performed with annealing at 60°C for 20 seconds and extension at 68°C for 20 seconds.

The proinsulin analog fragment obtained above was cloned into pET22b vector (Novagen). In order to express the proinsulin analog in the form of intracellular inclusion bodies, the pET22b vector was treated with the restriction enzymes *Ndel* and *Ba*mHI to remove signal sequences, the proinsulin analog PCR product was treated with the same restriction enzymes *Ndel* and *Bam*HI, and each isolated DNA was inserted into the pET22b cloning vector using T4DNA ligase. The expression vector obtained by the above result was named pET22b-InvPI.

### 2. Preparation of single-chain insulin analog (InvPI ΔRRKR)

In the nucleotide sequence (SEQ ID NO: 7) of the proinsulin analog obtained above, the first and second amino acids of the C peptide, arginine (1^{st} and 2^{nd} of SEQ ID NO: 3) and the last two amino acids of the C peptide (the 34^{th} and the 35^{th} amino acids of SEQ ID NO: 3) were removed using the following primers of SEQ ID NOS: 14 to 17.
5' GCTGGAGAACTACTGCAACGAGGCAGAGGACCTGCAGG 3' (forward direction, SEQ ID NO: 14)
5' CCTGCAGGTCCTCTGCCTCGTTGCAGTAGTTCTCCAGC 3' (reverse direction, SEQ ID NO: 15)
5' GGGGTCCCTGCAGTTTGTGAACCAACACCTGTGC 3' (forward direction, SEQ ID NO: 16)
5' GTTGGTTCACAAACTGCAGGGACCCCTCCAGGGC 3' (reverse direction, SEQ ID NO: 17)

The expression vector of the proinsulin analog obtained above was named pET22b-InvPI ΔRRKR, and the single-chain insulin analog was named InvPI ΔRRKR (SEQ ID NOS: 18 and 19).

**[Table 2]**

| | | | |
|---|---|---|---|
| Native proinsulin sequence and single-chain insulin analog sequence | | | |

| Analog | Sequence | | SEQ ID NO |
|---|---|---|---|
| Single-chain insulin analog (InvPIΔRRKR) | DNA | GGC ATT GTG GAA CAA TGC TGT ACC AGC ATC TGC TCC CTC TAC CAG CTG GAG AAC TAC TGC AAC GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG TTT GTG AAC CAA CAC CTG TGC GGC TCA CAC CTG GTG GAA GCT CTC TAC CTA GTG TGC GGG GAA CGA GGC TTC TTC TAC ACA CCC AAG ACC TAG | 18 |
| | Protein | GIVEQCCTSICSLYQLENYCNEAEDLQVG QVELGGGPGAGSLQPLALEGSLQFVNQH LCGSHLVEALYLVCGERGFFYTPKT | 19 |

### 3. Preparation of single-chain insulin analog inserted with binding protein sequence (FInvPI ΔRRKR) and its expression vector

For the purpose of helping protein expression in pET22b-InvPI ΔRRKR prepared in the above example and of facilitating N-terminal methionine deletion, the below primer was synthesized, in order to add a nucleotide sequence encoding the sequence of 10 amino acids (MATTSTATTR: SEQ ID NO: 20) to the 5' portion of the sequence of the InvPI ΔRRKR.

Specifically, for insertion of the sequence of 10 amino acids of SEQ ID NO: 20, a forward primer (SEQ ID NO: 21) was synthesized to comprise the initiation ATG codon, *Nde*l restriction enzyme site, and sequence of 10 amino acids, and a reverse primer (SEQ ID NO: 22) was synthesized to comprise the restriction enzyme *Ba*mHI sequence. The sequences of the above primers are as below.

5'		TTTAAGAAGGAGATATACATCATATGGCAACAACATCAACAGCAACT
ACGCGTGGCATTGTGGAACAATGCTG 3' (SEQ ID NO: 21)

5'
CAGCATTGTTCCACAATGCCACGCGTAGTTGCTGTTGATGTTGTTGCCATATGATGTAT
ATCTCCTTCTTAAA 3' (SEQ ID NO: 22)

Using the above primers, PCR was performed with annealing at 60°C for 30 seconds and extension at 68°C for 30 seconds for amplification of the single-chain insulin analog into which the binding protein was inserted.

The single-chain insulin analog fragment into which the binding protein obtained above was inserted was cloned into pET22b vector (Novagen). In order to express the single-chain insulin analog in the form of intracellular inclusion bodies, the pET22b vector was treated with the restriction enzymes *Nde*I and *Ba*mHI to remove the signal sequence, and the single-chain insulin analog PCR product with the binding protein inserted was treated with the same restriction enzymes *Nde*I and *Bam*HI, and each isolated DNA was inserted into the pET22b cloning vector using T4 DNA ligase. The expression vector obtained by the above result was named pET22b-FInvPI ΔRRKR, and the single-chain insulin analog into which the binding protein was inserted was named FInvPI ΔRRKR (Fusion-A-C-B RRKR; SEQ ID NOS: 23 and 24).

**[Table 3]**

| Single-chain insulin analog (FInvPI ΔRRKR)) sequence with binding protein inserted | | | |
|---|---|---|---|
| Analog | Sequence | | SEQ ID NO |
| Binding protein single-chain insulin | DNA | ATG GCA ACA ACA TCA ACA GCA ACT ACG CGT GGC ATT GTG GAA CAA TGC TGT ACC AGC ATC TGC TCC CTC TAC CAG CTG GAG AAC TAC TGC AAC GAG GCA GAG GAC CTG | 23 |
| analog (Fusion-A-C-B ΔRRKR) | | CAG GTG GGG CAG GTG GAG CTG GGC GGG GGC CCT GGT GCA GGC AGC CTG CAG CCC TTG GCC CTG GAG GGG TCC CTG CAG TTT GTG AAC CAA CAC CTG TGC GGC TCA CAC CTG GTG GAA GCT CTC TAC CTA GTG TGC GGG GAA CGA GGC TTC TTC TAC ACA CCC AAG ACC TAG | |
| | Protein | MATTSTATTRGIVEQCCTSICSLYQLENYCNEAE DLQVGQVELGGGPGAGSLQPLALEGSLQFVNQ HLCGSHLVEALYLVCGERGFFYTPKT | 24 |

The pET22b-FInvPI ΔRRKR expression vector encodes the amino acid sequence of SEQ ID NO: 24 under the control of T7 promoter, and the proinsulin analog protein was expressed in the form of inclusion bodies in the host cell.

### Example 2: Expression of single-chain insulin analog

The expression vector constructed in Example 1 above was used to express a recombinant single-chain insulin analog under the control of T7 promoter. *E. coli* BL21DE3 (*E. coli* B F-dcm ompT hsdS(rB-mB-) gal λ(DE3) Novagen) was transformed with each recombinant single-chain insulin analog expression vector. For the transformation method, a method recommended by Novagen was used. Each transformed single colony transformed with each recombinant expression vector was collected, inoculated in 2X Luria Broth medium containing ampicillin (50 µg/mL) and incubated at 37°C for 15 hours. Recombinant strain cultures and 2X LB medium containing 30% glycerol were mixed at a ratio of 1:1 (v/v), and each 1 mL was dispensed into cryo-tubes and stored at -140°C. The resultant was used as a cell stock for the production of recombinant fusion proteins.

For the expression of recombinant single-chain insulin analogs, one vial of each cell stock was dissolved and inoculated in 500 mL of 2X LB, followed by shake culturing at 37°C for 14 hours to 16 hours. When the value of OD 600 nm reached 4.0 or more, the culture was finished, and the resultant was used as the seed culture solution. Using a 5L fermenter (Bioflo-320, NBS, USA), seed cultures were inoculated into 1.6 L of fermentation medium and initial fermentation was started. The culture condition was maintained at a pH of 6.70, using a temperature of 37°C, air volume of 2.0 L/min (1 vvm), stirring rate of 650 rpm, and 30% ammonia water. For fermentation process, when the nutrients in the culture were limited, an additional medium (feeding solution) was added to proceed with fed-batch culture. The growth of the strains was monitored by OD values, and IPTG with a final concentration of 500 µM was introduced at OD values of 70 or more. The culture was further progressed up to about 23 hours to about 25 hours after the introduction of IPTG thereto, and after completion of the culture, the recombinant strains were harvested using a centrifuge and stored at -80°C until use.

### Example 3: Extraction and refolding of recombinant single-chain insulin analog

Cells were crushed and refolded from the single-chain insulin analog expressing *E. coli* obtained in the above example to convert the single-chain insulin analog into a soluble form. Cell pellets corresponding to 1 L of culture were suspended in 1 L of a lysis buffer solution (20 mM Tris-HCl pH 9.0, 1 mM EDTA pH 8.0, 0.2 M NaCl, 0.5% Triton X-100), and recombinant *E. coli* was crushed at 15,000 psi using a microfludizer. After centrifugation for 30 minutes at 12,000 g, the supernatant was discarded, and the pellet was washed with 1 L of a lysis buffer solution (50 mM Tris-HCl pH 9.0, 1 mM EDTA pH 9.0, 0.2 M NaCl, 0.5% Triton X-100). After centrifugation under the same conditions as above, the supernatant was discarded, and the pellet was resuspended with distilled water. After centrifugation under the same conditions, a washed *E. coli* inclusion body pellet was obtained. The washed inclusion pellet was resuspended in 1 L of a solubilization buffer solution (6 M Urea, 15 mM Glycine, pH 10.6) and was stirred at room temperature for 3 hours. For reduction of the solubilized single-chain insulin analog, L-Cysteine-HCl at a final concentration of 15 mM was introduced thereto and the mixture was stirred at room temperature for 1.5 hours. The refolding process was performed by mixing the reduced single-chain insulin analog with 3 L of 95 mM Glycine pH 10.5 solution at 4°C and stirring for 40 hours to 70 hours. A reaction stop solution (1 M Na-Cit pH 2.0:acetic acid = 1:1.67 (v/v)) corresponding to 7% of the solution volume was added to terminate the refolding reaction. The suspension was removed by centrifugation at 12,000 g for 30 minutes, followed by passing through a 0.45 µm filter to remove additional suspension.

### Example 4: Primary cation column chromatography

The single-chain insulin analog refolding solution obtained in Example 3 above was purified by applying to a cationic SP FF (GE Healthcare) column. The column was equilibrated with a binding buffer solution (20 mM Na-Citrate pH 3.0, 45% ethanol) prior to introduction of the refolding solution, and 4-column volumes of an elution buffer solution (20 mM Na-Citrate pH 3.0, 0.5 M KCl, 45% ethanol) was flown at a gradient of 0% to 100% for elution.

### Example 5: Removal of binding protein using protease

In order to facilitate protein expression, a fusion protein is present at the N-terminus of the single-chain insulin analog. To remove this binding protein, trysin (Roche), which is a protease, was used. Sephadex G-25 (GE Healthcare) column was used to replace the eluate obtained in cationic column chromatography with a buffer for enzyme reaction (50 mM Tris-HCl pH 8.0). After treating 163.28 units of trypsin per gram of protein eluted from the column, the reaction was performed at 15°C for 15 hours. In order to stop the removal reaction after 15 hours, buffer replacement was performed using a column with 20 mM Na-phosphate buffer solution (pH 2.0).

### Example 6: Secondary cation column chromatography

SP HP (GE Healthcare) column was used for the purification of the single-chain insulin analog from which the binding protein was removed. The column was equilibrated with a binding buffer solution (20 mM Na-Citrate pH 3.0, 45% ethanol), and 8-column volumes of an elution buffer solution (20 mM Na-Citrate pH 3.0, 0.5 M KCl, 45% ethanol) was flown at a gradient of 0% to 56% for elution.

### Example 7: Reverse phase chromatography

Source 30RPC (GE Healthcare) was used to further purify the protein eluted in the cation column chromatography of Example 6 with a reverse phase column. To prepare a buffer solution to be used in the purification process, a basic buffer solution, 0.05 M Na-phosphate, 0.1M Na-percholate (pH 2.3) was prepared and filtered by a 0.22 µm filter (Sartorious). The binding buffer solution was prepared by mixing 90% of the basic buffer solution with 10% of isopropyl alcohol (w/v), and the elution buffer solution was prepared by mixing 55% of the basic buffer solution and 45% isopropyl alcohol. 12.5-column volume of the elution buffer solution was flown at a gradient of 0% to 100% for elution. The eluted samples were measured for purity by RP-HPLC (C18, C4) and SE-HPLC analysis. The results of purity measurement are shown in Table 2.

### Example 8: Pegylation of single-chain insulin analog

The single-chain insulin analog that was purified to high purity by the process of Example 7 above was buffer-exchanged with 100 mM potassium phosphate (pH 6.0) to adjust the concentration to 5 mg/mL, and in order to pegylate 3.4 kDa PropionylALD2 PEG to the N-terminus of the single-chain insulin analog, PEG was added at a molar ratio of the single-chain insulin analog to PEG of 1:10 and dissolved completely. Then, sodium cyanoborohydride (NaCNBH₃), which is a reducing agent, was added to a concentration of 20 mM and reacted at room temperature for 70 minutes. Then, the resultant was diluted 10-fold with 20 mM sodium citrate (pH 2.0) buffer containing 30% ethanol and was injected into a Source S column (15 mm/15.5 cm, GE Healthcare). The Source S column was a column that was equilibrated with 20 mM sodium citrate (pH 2.0 q) buffer containing 30% ethanol, and the flow rate was set at 2.5 mL/min. In order to purify one pegylated single-chain insulin analog, one pegylated single-chain insulin analog was purified by flowing 10-column volumes of 20 mM sodium citrate, pH 2.0, 0.25 M KCl buffer containing 30% ethanol to a concentration of 0% to 100%.

### Example 9: Preparation of single-chain insulin analog

The pegylated single-chain insulin analog obtained using the method of Example 8 above was coupled with an immunoglobulin Fc. The molar ratio of a single-chain insulin analog to immunoglobulin Fc was set at 1:4, and the total protein concentration was set at 30 mg/mL to react at 4°C for 18 hours. In this case, sodium cyanoborohydride (NaCNBH₃) was added as a reducing agent to a concentration of 20 mM. Then, the buffer was exchanged with a 20 mM Tris (pH 7.5) buffer using a Sephadex G25 column, and then injected into a SourceQ column (25 mm/17.6 cm, GE Healthcare). The Source Q column was a column that was equilibrated with 20 mM Tris (pH 7.5) buffer, and the flow rate was set at 7 mL/min. In order to elute the coupling protein, 20 mM Tris, pH 7.5, 0.5M NaCl buffer was flown by 10-column volume linearly from 0% to 30% for elution. Unreacted immunoglobulin Fc could be removed with a Source Q column. 1.5 M ammonium sulfate was added to the coupling protein solution eluted in the Source Q and the mixture was injected into a Source Iso column (25 mm/12.3 cm, GE Healthcare). The Source Iso column was a column that was equilibrated with 20 mM Tris-HCl (pH 7.5), 1.5 M ammonium sulfate buffer, and the flow rate was set at 7.5 mL/min. In order to purify the single-chain insulin analog-PEG-immunoglubilin Fc, 37-column volumes of 20 mM Tris-HCl (pH 7.5) buffer was linearly flown at a concentration from 0% to 100%.

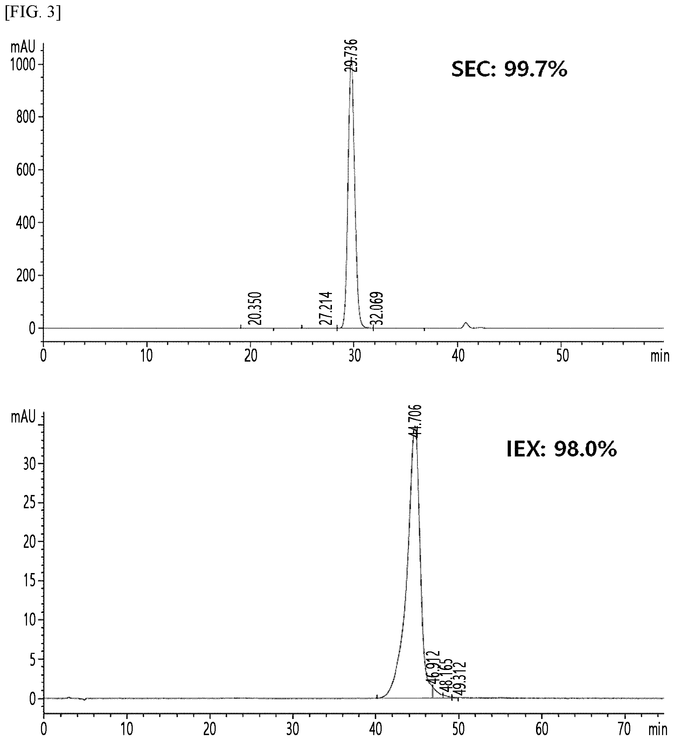

The final purified single-chain insulin analog conjugate (single-chain insulin analog-PEG-immunoglobulin Fc) in the Source Iso column was analyzed by SE-HPLC and IE-HLPC and the results are shown in FIG. 3.

### Example 10: Confirmation of insulin receptor binding affinity of single-chain insulin analog and single-chain insulin analog conjugate

In order to measure the insulin receptor binding affinity of the single-chain insulin analog, the analysis was performed using the Scintillation Proximity Assay (SPA) method. In a 96 well pico-plate, the plasma membrane of CHO cell line in which insulin receptors were expressed and PVT SPA beads were placed together. In order to confirm the binding affinity to insulin receptors, human insulin and each insulin analog diluted to 10 or more different concentrations, and insulin attached with 125 iodine which is a radioactive isotope as a competitor were placed together and were subjected to a competition reaction at room temperature for 4 hours. After 4 hours, the binding affinity of the insulin receptors was measured using a beta counter. The binding affinity of each material was calculated by IC50 using GraphPad Prism 6 software, and it was quantified by the insulin receptor binding affinity of the single-chain insulin analog relative to the insulin receptor binding affinity of native insulin.

As a result, compared to native insulin, the binding affinities of 69% of the single-chain insulin analog and 1.9% of the single-chain insulin analog conjugate were confirmed, respectively (Table 4). As described above, the single-chain insulin analog of the present invention showed a reduced insulin receptor binding affinity compared to native insulin.

**[Table 4]**

| Material | | Insulin receptor binding affinity (vs. natural insulin) |
|---|---|---|
| Single-chain insulin analog | Single-chain insulin analog | 90% |
| Immunoglobulin conjugate | Native insulin conjugate | 4.6% |
| | Single-chain insulin analog conjugate | 1.0% |

### Example 11: Confirmation of in vitro efficacy of single-chain insulin analog conjugate

In order to measure the *in vitro* efficacy of a single-chain insulin analog conjugate, glucose uptake (or lipid synthesis) test was carried out using a mouse-derived 3T3-L1 cell line differentiated into adipocytes. 3T3-L1 cells were subcultured 2 to 3 times a week using Dulbeco's Modified Eagle's Medium (DMEM, Gibco, Cat. No. 12430) including 10% newborn calf serum (NBCS). 3T3-L1 cells were suspended using a differentiation medium (DMEM including 10% FBS), and then inoculated to a density of 5 × 10⁴ cells per hole in a 48-hole plate and cultured for 48 hours. For differentiation into adipocytes, 1 µg/mL human insulin (Sigma, Cat. No. 19278), 0.5 µM 3-isobutyl-1-methylxanthine (IBMX, Sigma, Cat. No. 15879), and 1 µM dexamethasone (Sigma, Cat. No. D4902) were mixed in a medium for differentiation, and was added in an amount of 250 µL per hole after removing the existing medium. After 48 hours, it was again replaced with a medium in which only 1 µg/mL of human insulin was added to a medium for differentiation. Then, the induction of differentiation into adipocytes was confirmed for 12 days while replacing the medium for differentiation including 1 µg/mL of human insulin every 48 hours. For the glucose uptake test, the cells, which completed differentiation, were washed once with serum-free DMEM medium, followed by placing by 250 µL each to induce serum depletion for 4 hours. Human insulin was prepared by a 10-fold serial dilution with serum-free DMEM medium from 10 µM to 0.001 nM, and the single-chain insulin-immunoglobulin Fc conjugate was prepared by a 10-fold serial dilution with serum-free DMEM medium from 20 µM to 0.002 nM. 250 µL each of the thus prepared samples was added to the cells, and then cultured in a 37°C, 5% CO₂ incubator for 24 hours. In order to measure the glucose residual amount of a cultured medium, 200 µL of the medium was collected and diluted 5 times with D-PBS, respectively, and GOPOD Assay Kit (GOPOD, Megazyme, Cat. No. K-GLUC) analysis was performed. Based on the absorbance of the glucose standard solution, the residual glucose concentration of the medium was calculated, and ECso for glucose uptake was calculated, respectively.

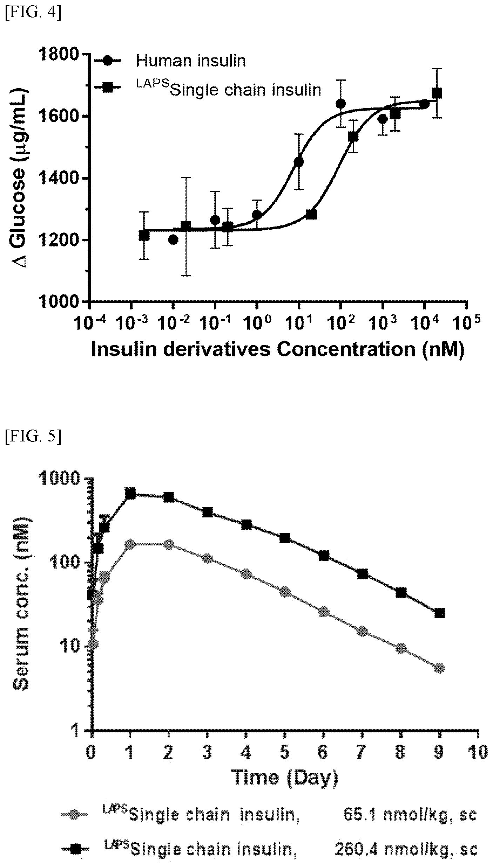

As a result, it was confirmed that the single-chain insulin analog conjugate (single-chain insulin analog-PEG-immunoglobulin Fc) had a glucose uptake of 8.1% (Table 5, FIG. 4) compared to human insulin.

**[Table 5]**

| | | |
|---|---|---|
| Glucose uptake of single-chain insulin analog | | |

| Material | EC₅₀ (nM) | % *vs*. Human insulin |
|---|---|---|
| Natural insulin | 7.5 | 100 |
| Single-chain insulin analog conjugate | 92.8 | 8.1 |

### Example 12: Pharmacokinetics confirmation of single-chain insulin analog conjugate

In order to confirm the pharmacokinetics of a single-chain insulin analog conjugate, blood concentration comparison test was conducted in normal rats (SD rat, male, 6 weeks old) over time. The single-chain insulin analog conjugate was subcutaneously administered in an amount of 65.1 nmol/kg and 260.4 nmol/kg, respectively, and the blood was collected at 0, 1, 4, 8, 24, 48, 72, 96, 120, 144, 168, 192, and 216 hours, respectively. The residual concentrations in the blood of the single-chain insulin analog conjugate at each time were measured using the enzyme linked immunosorbent assay (ELISA), and for the kit used was the Insulin ELISA (ALPCO, USA). As a detection antibody, a mouse anti-human IgG4 HRP conjugate (Alpha Diagnostic Intl, Inc, USA) was used.

As a result of observing the pharmacokinetics of the single-chain insulin analog conjugate, the half-life of the single-chain insulin analog conjugate was 31 hours to 32 hours, which is about 7 times longer than the reported half-life (4.3 hours) of insulin glargine in rats, which is a long-acting insulin analog currently on the market (FIG. 5).

These results suggest that when the single-chain insulin analog of the present invention, which was modified to reduce insulin receptor binding affinity, forms a conjugate with an immunoglobulin Fc region, the actual *in vivo* blood half-life can be dramatically increased to be provided as a stable insulin formulation and can be effectively used as a diabetes treatment. Moreover, these results also suggest that the single-chain insulin analog itself according to the present invention, even when combined with a variety of carriers, can exhibit the same effect by having a reduced binding affinity with the insulin receptors and a reduced potency.

From the above description, those skilled in the art will appreciate that the present invention can be implemented in other specific forms without changing the technical spirit or essential features. In this regard, the embodiments described above are to be understood in all respects as illustrative and not restrictive. The scope of the present invention should be construed that all changes or modifications derived from the meaning and scope of the following claims and equivalent concepts rather than the detailed description are included in the scope of the present invention.

## Claims

1. A single-chain insulin analog conjugate having Chemical Formula 1 below:
[Chemical Formula 1] X-Y-Z-La-F
wherein:
X is native insulin A chain, B chain, or an analog thereof,
Y is native insulin C-peptide or an analog thereof,
Z is native insulin B chain, A chain, or an analog thereof,
L is a linker,
A is 0 or a natural number, with the proviso that when a is 2 or more, each L is independent of each other,
F is a material capable of increasing an *in vivo* half-life of an insulin analog,
with the proviso that cases where X, Y, and Z are a B chain, C-peptide, and A chain of native insulin, respectively, or an A chain, C-peptide, and B chain of native insulin, respectively, are excluded, and
X-Y-Z forms a single-chain insulin analog.

2. The single-chain insulin analog conjugate of claim 1, wherein the single-chain insulin analog is an analog, a variant, or a fragment thereof, which is modified by at least one method selected from substitution, addition and modification of at least one amino acid, and change in arrangement of order of insulin A chain, C-peptide, and B-chain, compared to native proinsulin.

3. The single-chain insulin analog conjugate of claim 1 or 2, wherein the single-chain insulin analog is a single-chain insulin analog in which X, Y, and Z are each linked by a linker.

4. The single-chain insulin analog conjugate according to any one of claims 1 to 3, wherein the analog of the C-peptide is an analog of the C-peptide in which at least one amino acid selected from the group consisting of the 1^{st} amino acid, the 2^{nd} amino acid, the 34^{th} amino acid, and the 35^{th} amino acid of native insulin C-peptide is substituted with another amino acid or deleted.

5. The single-chain insulin analog conjugate according to any one of claims 1 to 4, wherein a material capable of increasing an *in vivo* half-life of an insulin analog is selected from the group consisting of polyethylene glycol, fatty acid, cholesterol, albumin and a fragment thereof, an albumin-binding material, a polymer of repeating units of a specific amino acid sequence, an antibody, an antibody fragment, an FcRn-binding material, *in vivo* connective tissue, nucleotide, fibronectin, transferrin, saccharide, and a polymer.

6. The single-chain insulin analog conjugate according to any one of claims 1 to 5, wherein L is selected from the group consisting of peptide, polyethylene glycol, fatty acid, saccharide, polymer, low molecular weight compound, nucleotide, and a combination thereof.

7. The single-chain insulin analog conjugate according to any one of claims 1 to 6, wherein X-Y-Z and F are linked with each other via L by a covalent chemical bond, a non-covalent chemical bond, or a combination thereof.

8. The single-chain insulin analog conjugate of claim 6, wherein the polymer is selected from the group consisting of polypropylene glycol, ethylene glycol-propylene glycol copolymer, polyoxyethylated polyol, polyvinyl alcohol, polysaccharide, dextran, polyvinyl ethyl ether, biodegradable polymer, lipid polymer, chitin, hyaluronic acid, oligonucleotide, and a combination thereof.

9. The single-chain insulin analog conjugate of claim 5, wherein the FcRn binding material is an immunoglobulin Fc region.

10. The single-chain insulin analog conjugate of claim 9, wherein the immunoglobulin Fc region is aglycosylated.

11. The single-chain insulin analog conjugate of claim 9 or 10, wherein the immunoglobulin Fc region is composed of 1 to 4 domains selected from the group consisting of CH1, CH2, CH3, and CH4 domains.

12. The single-chain insulin analog conjugate according to any one of claims 9 to 11, wherein the immunoglobulin Fc region is an Fc region derived from IgG, IgA, IgD, IgE, or IgM.

13. The single-chain insulin analog conjugate of claim 12, wherein each domain of the immunoglobulin Fc region is a hybrid of domains with different origins derived from an immunoglobulin selected from the group consisting of IgG, IgA, IgD, IgE, and IgM.

14. The single-chain insulin analog conjugate according to any one of claims 9 to 12, wherein the immunoglobulin Fc region is a dimer or multimer consisting of single-chain immunoglobulins composed of domains of the same origin.

15. The single-chain insulin analog conjugate according to any one of claims 9 to 14, wherein the immunoglobulin Fc region further comprises a hinge region.

16. The single-chain insulin analog conjugate of claim 9, wherein the immunoglobulin Fc region is an IgG4 Fc region.

17. The single-chain insulin analog conjugate of claim 9, wherein the immunoglobulin Fc region is a human aglycosylated IgG4 Fc region.

18. A single-chain insulin analog having Chemical Formula 2 below:
[Chemical Formula 2] X-Y-Z
wherein:
X is native insulin A chain, B chain, or an analog thereof,
Y is native insulin C-peptide or an analog thereof,
Z is native insulin B chain, A chain, or an analog thereof, and
with the proviso that cases where X, Y, and Z are B chain, C-peptide, and A chain of native insulin, respectively, or A chain, C-peptide, and B chain of native insulin, respectively, are excluded.

19. The single-chain insulin analog of claim 18, wherein the analog of the C-peptide is an analog of the C-peptide in which at least one amino acid selected from the group consisting of the 1^{st} amino acid, the 2^{nd} amino acid, the 34^{th} amino acid, and the 35^{th} amino acid of native insulin C-peptide is substituted with another amino acid or deleted.

20. A long acting single-chain insulin formulation with enhanced *in vivo* duration and stability, comprising:
the single-chain insulin analog conjugate according to any one of claims 1 to 17; or
the single-chain insulin analog of claim 18 or 19.

21. A pharmaceutical composition for preventing or treating diabetes, comprising:
the single-chain insulin analog conjugate according to any one of claims 1 to 17; or
the single-chain insulin analog of claim 18 or 19.

22. A method for preparing the single-chain insulin analog conjugate according to any one of claims 1 to 17, comprising linking the single-chain insulin analog of claim 18 or 19 to a material capable of increasing the *in vivo* half-life thereof.

23. The method of claim 22, wherein the single-chain insulin analog and the material capable of increasing the *in vivo* half-life thereof are linked via a linker.

24. The method of claim 23, wherein the linker is a non-peptidyl linker having a reactive group selected from the group consisting of an aldehyde group, a maleimide group, and a succinimide derivative.

25. The method of claim 24, wherein the succinimide derivative is succinimidyl carboxymethyl, succinimidyl valerate, succinimidyl methylbutanoate, succinimidyl methylpropionate, succinimidyl butanoate, succinimidyl propionate, N-hydroxysuccinimide, or succinimidyl carbonate.

26. The method according to any one of claims 22 to 25, wherein the single-insulin analog is obtained by a method comprising (a) and (b) below:
(a) expressing a single-chain insulin analog in a form, in which a peptide consisting of 5 to 20 amino acids comprising a protease cleavage site is fused to an N-terminus of the single-chain insulin analog; and
(b) removing the peptide fused to the single-chain insulin analog.

27. An isolated nucleic acid encoding the single-chain insulin analog of claim 18 or 19.

28. A recombinant expression vector comprising the nucleic acid of claim 27.

29. A transformant comprising the recombinant expression vector of claim 28.

30. A method for preparing the single-chain insulin analog of claim 18 or 19, comprising:
a) expressing a single-chain insulin analog by culturing a transformant comprising a nucleic acid encoding the single-chain insulin analog of claim 18 or 19; and
b) isolating and purifying the expressed single-chain insulin analog.


<110>     HANMI PHARM. CO., LTD.

<120>     A long acting single chain insulin analog and a conjugate thereof

<130>     OPA18290-PCT

<150>     KR 10-2017-0126476
<151>     2017-09-28

<160>     24

<170>     KoPatentIn 3.0

<210>     1
<211>     21
<212>     PRT
<213>     Homo sapiens

<400>     1
Gly Ile Val Glu Gln Cys Cys Thr Ser Ile Cys Ser Leu Tyr Gln Leu
  1               5                  10                  15

Glu Asn Tyr Cys Asn
             20



<210>     2
<211>     30
<212>     PRT
<213>     Homo sapiens

<400>     2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr
             20                  25                  30



<210>     3
<211>     35
<212>     PRT
<213>     Homo sapiens

<400>     3
Arg Arg Glu Ala Glu Asp Leu Gln Val Gly Gln Val Glu Leu Gly Gly
  1               5                  10                  15

Gly Pro Gly Ala Gly Ser Leu Gln Pro Leu Ala Leu Glu Gly Ser Leu
             20                  25                  30

Gln Lys Arg
         35



<210>     4
<211>     261
<212>     DNA
<213>     Homo sapiens

<400>     4
tttgtgaacc aacacctgtg cggctcacac ctggtggaag ctctctacct agtgtgcggg        60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaacta g                                               261

<210>    5
<211>    86
<212>    PRT
<213>    Homo sapiens

<400>    5
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>    6
<211>    261
<212>    DNA
<213>    Artificial Sequence

<220>
<223>    single chain insulin analog (A-C-B)

<400>    6
ggcattgtgg aacaatgctg taccagcatc tgctccctct accagctgga gaactactgc      60

aaccgccggg aggcagagga cctgcaggtg gggcaggtgg agctgggcgg gggccctggt     120

gcaggcagcc tgcagccctt ggccctggag gggtccctgc agaagcgttt tgtgaaccaa     180

cacctgtgcg gctcacacct ggtggaagct ctctacctag tgtgcgggga acgaggcttc     240

ttctacacac ccaagaccta g                                               261

<210>    7
<211>    86
<212>    PRT
<213>    Artificial Sequence

<220>
<223>    single chain insulin analog (A-C-B)

<400>    7
Gly Ile Val Glu Gln Cys Cys Thr Ser Ile Cys Ser Leu Tyr Gln Leu
1               5                   10                  15

Glu Asn Tyr Cys Asn Arg Arg Glu Ala Glu Asp Leu Gln Val Gly Gln
            20                  25                  30

Val Glu Leu Gly Gly Gly Pro Gly Ala Gly Ser Leu Gln Pro Leu Ala
        35                  40                  45

Leu Glu Gly Ser Leu Gln Lys Arg Phe Val Asn Gln His Leu Cys Gly
    50                  55                  60

Ser His Leu Val Glu Ala Leu Tyr Leu Val Cys Gly Glu Arg Gly Phe
65                  70                  75                  80

Phe Tyr Thr Pro Lys Thr
                85


<210>    8
<211>    34
<212>    DNA
<213>    Artificial Sequence

<220>
<223>    forward primer for A chain of proinsulin analog


<400>    8
ggaattccat atgggcattg tggaacaatg ctgt                                34


<210>    9
<211>    34
<212>    DNA
<213>    Artificial Sequence

<220>
<223>    reverse primer for A chain of proinsulin analog


<400>    9
ctgcctcccg gcggttgcag tagttctcca gctg                                34


<210>    10
<211>    34
<212>    DNA
<213>    Artificial Sequence

<220>
<223>    forward primer for C peptide of proinsulin analog


<400>    10
gaactactgc aaccgccggg aggcagagga cctg                                34


<210>    11
<211>    34
<212>    DNA

<213>    Artificial Sequence

<220>
<223>    reverse primer for C peptide of proinsulin analog


<400>    11
gttggttcac aaaacgcttc tgcagggacc cctc                                    34


<210>    12
<211>    34
<212>    DNA
<213>    Artificial Sequence

<220>
<223>    forward primer for B chain of proinsulin analog


<400>    12
cctgcagaag cgttttgtga accaacacct gtgc                                    34


<210>    13
<211>    34
<212>    DNA
<213>    Artificial Sequence

<220>
<223>    reverse primer for B chain of proinsulin analog


<400>    13
cgcggatccc taggtcttgg gtgtgtagaa gaag                                    34


<210>    14
<211>    38
<212>    DNA
<213>    Artificial Sequence

<220>
<223>    forward primer for single chain insulin analog (InvPI deltaRRKR)


<400>    14
gctggagaac tactgcaacg aggcagagga cctgcagg                                38


<210>    15
<211>    38
<212>    DNA
<213>    Artificial Sequence

<220>
<223>    forward primer for single chain insulin analog (InvPI deltaRRKR)


<400>    15
cctgcaggtc ctctgcctcg ttgcagtagt tctccagc                                38


<210>    16

<211>    34
<212>    DNA
<213>    Artificial Sequence

<220>
<223>    forward primer for single chain insulin analog (InvPI deltaRRKR)


<400>    16
ggggtccctg cagtttgtga accaacacct gtgc                                    34


<210>    17
<211>    34
<212>    DNA
<213>    Artificial Sequence

<220>
<223>    reverse primer for single chain insulin analog (InvPI deltaRRKR)


<400>    17
gttggttcac aaactgcagg gacccctcca gggc                                    34


<210>    18
<211>    249
<212>    DNA
<213>    Artificial Sequence

<220>
<223>    single chain insulin analog (InvPI deltaRRKR)


<400>    18
ggcattgtgg aacaatgctg taccagcatc tgctccctct accagctgga gaactactgc        60

aacgaggcag aggacctgca ggtggggcag gtggagctgg gcgggggccc tggtgcaggc       120

agcctgcagc ccttggccct ggaggggtcc ctgcagtttg tgaaccaaca cctgtgcggc       180

tcacacctgg tggaagctct ctacctagtg tgcggggaac gaggcttctt ctacacaccc       240

aagacctag                                                               249


<210>    19
<211>    82
<212>    PRT
<213>    Artificial Sequence

<220>
<223>    single chain insulin analog (InvPI deltaRRKR)


<400>    19
Gly Ile Val Glu Gln Cys Cys Thr Ser Ile Cys Ser Leu Tyr Gln Leu
1               5                   10                  15

Glu Asn Tyr Cys Asn Glu Ala Glu Asp Leu Gln Val Gly Gln Val Glu
            20                  25                  30

Leu Gly Gly Gly Pro Gly Ala Gly Ser Leu Gln Pro Leu Ala Leu Glu

35                  40                  45

Gly Ser Leu Gln Phe Val Asn Gln His Leu Cys Gly Ser His Leu Val
    50                  55                  60

Glu Ala Leu Tyr Leu Val Cys Gly Glu Arg Gly Phe Phe Tyr Thr Pro
65                  70                  75                  80

Lys Thr

<210>    20
<211>    10
<212>    PRT
<213>    Artificial Sequence

<220>
<223>    protease cleavage site

<400>    20
Met Ala Thr Thr Ser Thr Ala Thr Thr Arg
1               5                   10

<210>    21
<211>    73
<212>    DNA
<213>    Artificial Sequence

<220>
<223>    forward primer for single chain insulin analog (FInvPI deltaRRKR)

<400>    21
tttaagaagg agatatacat catatggcaa caacatcaac agcaactacg cgtggcattg        60

tggaacaatg ctg                                                           73

<210>    22
<211>    73
<212>    DNA
<213>    Artificial Sequence

<220>
<223>    reverse primer for single chain insulin analog (FInvPI deltaRRKR)

<400>    22
cagcattgtt ccacaatgcc acgcgtagtt gctgttgatg ttgttgccat atgatgtata        60

tctccttctt aaa                                                           73

<210>    23
<211>    279
<212>    DNA
<213>    Artificial Sequence

<220>
<223>    single chain insulin analog (FInvPI deltaRRKR)

<400>    23
atggcaacaa catcaacagc aactacgcgt ggcattgtgg aacaatgctg taccagcatc        60

tgctccctct accagctgga gaactactgc aacgaggcag aggacctgca ggtggggcag       120

gtggagctgg gcgggggccc tggtgcaggc agcctgcagc ccttggccct ggaggggtcc       180

ctgcagtttg tgaaccaaca cctgtgcggc tcacacctgg tggaagctct ctacctagtg       240

tgcggggaac gaggcttctt ctacacaccc aagacctag                              279


<210>    24
<211>    92
<212>    PRT
<213>    Artificial Sequence

<220>
<223>    single chain insulin analog (FInvPI deltaRRKR)


<400>    24
Met Ala Thr Thr Ser Thr Ala Thr Thr Arg Gly Ile Val Glu Gln Cys
1               5                   10                  15

Cys Thr Ser Ile Cys Ser Leu Tyr Gln Leu Glu Asn Tyr Cys Asn Glu
            20                  25                  30

Ala Glu Asp Leu Gln Val Gly Gln Val Glu Leu Gly Gly Gly Pro Gly
        35                  40                  45

Ala Gly Ser Leu Gln Pro Leu Ala Leu Glu Gly Ser Leu Gln Phe Val
    50                  55                  60

Asn Gln His Leu Cys Gly Ser His Leu Val Glu Ala Leu Tyr Leu Val
65                  70                  75                  80

Cys Gly Glu Arg Gly Phe Phe Tyr Thr Pro Lys Thr
                85                  90